(19) Europäisches Patentamt / European Patent Office / Office européen des brevets

(11) **EP 4 067 387 B1**

(12) **EUROPEAN PATENT SPECIFICATION**

(45) Date of publication and mention of the grant of the patent:
**28.01.2026 Bulletin 2026/05**

(21) Application number: **20892544.6**

(22) Date of filing: **25.11.2020**

(51) International Patent Classification (IPC):
*C07K 16/46* (2006.01)    *C12N 15/13* (2006.01)
*A61K 39/395* (2006.01)    *A61P 35/00* (2006.01)
*C07K 16/22* (2006.01)    *A61P 35/02* (2006.01)
*G01N 33/574* (2006.01)    *C07K 16/28* (2006.01)

(52) Cooperative Patent Classification (CPC):
**A61P 35/00; A61P 35/02; C07K 16/22;**
**C07K 16/2818; G01N 33/57407;** C07K 2317/24;
C07K 2317/31; C07K 2317/52; C07K 2317/524;
C07K 2317/732; C07K 2317/734; C07K 2317/92;
G01N 2333/475; G01N 2333/70532;
G01N 2800/7014

(86) International application number:
**PCT/CN2020/131447**

(87) International publication number:
**WO 2021/104302 (03.06.2021 Gazette 2021/22)**

(54) **ANTI-PD-1-ANTI-VEGFA BISPECIFIC ANTIBODY, PHARMACEUTICAL COMPOSITION AND USE THEREOF**

BISPEZIFISCHER ANTI-PD-1-ANTI-VEGFA-ANTIKÖRPER, PHARMAZEUTISCHE ZUSAMMENSETZUNG UND VERWENDUNG DAVON

ANTICORPS BISPÉCIFIQUE ANTI-PD-1-ANTI-VEGFA, COMPOSITION PHARMACEUTIQUE ET LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.11.2019 CN 201911164156**

(43) Date of publication of application:
**05.10.2022 Bulletin 2022/40**

(60) Divisional application:
**25222544.6**

(73) Proprietor: **Akeso Biopharma Co., Ltd.**
**Zhongshan, Guangdong 528437 (CN)**

(72) Inventors:
• **ZHANG, Peng**
**Zhongshan, Guangdong 528437 (CN)**
• **LI, Baiyong**
**Zhongshan, Guangdong 528437 (CN)**

• **XIA, Yu**
**Zhongshan, Guangdong 528437 (CN)**
• **WANG, Zhongmin**
**Zhongshan, Guangdong 528437 (CN)**

(74) Representative: **Gill Jennings & Every LLP**
**The Broadgate Tower**
**20 Primrose Street**
**London EC2A 2ES (GB)**

(56) References cited:
WO-A1-2009/058812    WO-A1-2018/223923
WO-A1-2019/154349    WO-A2-2004/099249
CN-A- 105 175 545    CN-A- 108 752 476
CN-A- 109 053 895    CN-A- 109 053 895
CN-A- 110 498 857    CN-A- 110 563 849
CN-A- 110 960 543

- **MARJAN HEZAREH ET AL: "Effector function activities of a panel of mutants of a broadly neutralizing antibody against human immunodeficiency virus type 1", JOURNAL OF VIROLOGY, THE AMERICAN SOCIETY FOR MICROBIOLOGY, US, vol. 75, no. 24, 1 December 2001 (2001-12-01), pages 12161 - 12168, XP002635226, ISSN: 0022-538X, DOI: 10.1128/ JVI.75.24.12161-12168.2001**
- **Y. HU, F. ZHANG, S. CHEN, L. LI, L. ZHAO: "P105 Computational Design of IgG-like Tetra-specific Antibody Targeting EGFR/VEGF/PD-1/CTLA-4 Against NSCLC", JOURNAL OF THORACIC ONCOLOGY, vol. 13, no. 12S, 1 December 2018 (2018-12-01), pages S1086 - S1087, XP055816499, DOI: 10.1016/j.jtho.2018.10.131**

Remarks:
    The complete document including Reference Table(s) and the Sequence Listing(s) can be downloaded from the EPO website

Remarks:

**Description**

TECHNICAL FIELD

The present invention belongs to the field of tumor treatment and immunobiology, and relates to an anti-PD-1-anti-VEGFA bispecific antibody, a pharmaceutical composition thereof and use thereof. Specifically, the present invention relates to an anti-human PD-1-anti-human VEGFA bispecific antibody, a pharmaceutical composition thereof and use thereof.

BACKGROUND

Tumor, especially a malignant tumor, is a serious health-threatening disease in the world today, and it is the second leading cause of death among various diseases. In recent years, the incidence of the disease has been increasing remarkably. Malignant tumor is characterized by poor treatment response, high late metastasis rate and poor prognosis. Although conventional treatment methods (such as radiotherapy, chemotherapy and surgical treatment) adopted clinically at present alleviate the pain to a great extent and prolong the survival time, the methods have great limitations, and it is difficult to further improve their efficacy.

There are two distinct stages of tumor growth, namely, from a slow growth stage without blood vessels to a rapid proliferation stage with blood vessels. The angiogenesis enables the tumor to acquire enough nutrition to complete the blood vessel switching stage, and if there is no angiogenesis, the primary tumor will be no more than 1-2 mm, and thus the metastasis cannot be realized.

Vascular Endothelial Growth Factor (VEGF) is a growth factor which can promote division and proliferation of endothelial cells, promote formation of new blood vessels and improve blood vessel permeability, and it binds to vascular endothelial growth factor receptors on the cell surface and plays a role by activating tyrosine kinase signal transduction pathways. In tumor tissues, tumor cells, and macrophages and mast cells invading into tumors can secrete high-level VEGF, stimulate tumor vascular endothelial cells in a paracrine form, promote proliferation and migration of endothelial cells, induce angiogenesis, promote continuous growth of tumor, improve vascular permeability, cause fibrin deposition in surrounding tissues, and promote infiltration of mononuclear cells, fibroblasts and endothelial cells, which facilitates formation of tumor stroma and entry of tumor cells into new blood vessels, and promote tumor metastasis.

Therefore, inhibiting tumor angiogenesis is considered to be one of the most promising tumor treatment methods at present. The VEGF family includes: VEGFA, VEGFB, VEGFC, VEGFD and PIGF. Vascular Endothelial Growth Factor Receptors (VEGFRs) include VEGFR1 (also known as Flt1), VEGFR2 (also known as KDR or Flk1), VEGFR3 (also known as Flt4), and Neuropilin-1 (NRP-1). The first three receptors are similar in structure, belong to a tyrosine kinase superfamily, and are composed of an extramembrane region, a transmembrane segment and an intramembrane region, where the extramembrane region is composed of an immunoglobulin-like domain, and the intramembrane region is a tyrosine kinase region. VEGFR1 and VEGFR2 are located primarily on the surface of vascular endothelial cells, and VEGFR3 is located primarily on the surface of lymphatic endothelial cells.

Molecules of the VEGF family have different affinities for these receptors. VEGFA mainly acts in combination with VEGFR1, VEGFR2 and NRP-1. VEGFR1 is the earliest found receptor and has a higher affinity for VEGFA than VEGFR2 under normal physiological conditions, but it has a lower tyrosinase activity in intracellular segment than VEGFR2 (Ma Li, Chinese Journal of Birth Health and Heredity, 2016, 24 (5): 146-148).

VEGFR2 is the primary regulator of angiogenesis and vascular engineering, and has a much higher tyrosine kinase activity than VEGFR1. VEGFR2, after binding to ligand VEGFA, mediates the proliferation, differentiation and the like of vascular endothelial cells, as well as the formation process of blood vessels and the permeability of blood vessels (Roskoski R Jr. et al., Crit Rev Oncol Hematol, 2007, 62(3): 179-213). VEGFA, after binding to VEGFR2, mediates the transcriptional expression of intracellular related protein genes through the downstream PLC-$\gamma$-PKC-Raf-MEK-MAPK signaling pathway, and thus promotes the proliferation of vascular endothelial cells (Takahashi T et al., Oncogene, 1999, 18(13): 2221-2230).

VEGFR3 is one of the tyrosine kinase family members, and mainly expresses embryonic vascular endothelial cell and adult lymphatic endothelial cells, and VEGFC and VEGFD bind to VEGFR3 to stimulate proliferation and migration of lymphatic endothelial cells and promote neogenesis of lymphatic vessels; NRP-1 is a non-tyrosine kinase transmembrane protein and is incapable of independently transducing biological signals, and it is able to mediate signaling only after forming a complex with a VEGF tyrosine kinase receptor. (Ma Li, Chinese Journal of Birth Health and Heredity, 2016, 24 (5): 146-148).

VEGFA and VEGFR2 are mainly involved in regulation of angiogenesis, where before and after the binding of VEGFA to VEGFR2, a cascade reaction of numerous intermediate signals in upstream and downstream pathways is formed, and finally the physiological functions are changed by proliferation, survival, migration, permeability increase and infiltration to peripheral tissues, etc. of endothelial cells (Dong Hongchao et al., Journal of Modern Oncology, Sep. 2014, 22 (9): 2231-3).

Currently, there are several humanized monoclonal antibodies targeting human VEGF, particularly VEGFA, such as bevacizumab, which has been approved by the U.S. Food and Drug Administration for the treatment of various tumors such as non-small cell lung cancer, renal cell carcinoma, cervical cancer, and metastatic colorectal cancer in succession during 2004.

The programmed cell death receptor-1 (PD-1), also known as CD279, is a type I transmembrane glycoprotein membrane surface receptor, belongs to the CD28 immunoglobulin superfamily, and is commonly expressed in T cells, B cells, and myeloid cells. PD-1 has two natural ligands, PD-L1 and PD-L2. Both PD-L1 and PD-L2 belong to the B7 superfamily and are expressed constitutively or inducibly on the membrane surface of a variety of cells, including nonhematopoietic cells and a variety of tumor cells. PD-L1 is mainly expressed on T cells, B cells, DC and microvascular endothelial cells and a variety of tumor cells, while PD-L2 is expressed only on antigen presenting cells such as dendritic cells and macrophages. The interaction between PD-1 and its ligands can inhibit the activation of lymph, the proliferation of T cells, and the secretion of cytokines such as IL-2 and IFN-y.

A large number of research show that a tumor microenvironment can protect tumor cells from being damaged by immune cells, expression of PD-1 in lymphocytes infiltrated in the tumor microenvironment is up-regulated, and various primary tumor tissues are PD-L1 positive in immunohistochemical analysis, such as lung cancer, liver cancer, ovarian cancer, skin cancer, colon cancer and glioma. Meanwhile, the expression of PD-L1 in the tumor is significantly correlated with poor prognosis of cancer patients. Blocking the interaction between PD-1 and its ligands can promote the tumor-specific T cell immunity and enhance the immune elimination efficiency of tumor cells. A large number of clinical trials show that antibodies targeting PD-1 or PD-L1 can promote infiltration of CD8+ T cells into tumor tissues and up-regulate anti-tumor immune effector factors such as IL-2, IFN-y, granzyme B and perforin, thereby effectively inhibiting the growth of tumors.

In addition, anti-PD-1 antibodies may also be used in the treatment of viral chronic infections. Viral chronic infections are often accompanied by a loss of function of virus-specific effector T cells and a reduction in its number. The interaction between PD-1 and PD-L1 can be blocked by injecting a PD-1 antibody, thereby effectively inhibiting the exhaustion of effector T cells in viral chronic infection.

Due to the broad anti-tumor prospect and surprising efficacy of PD-1 antibodies, it is widely accepted in the industry that antibodies targeting the PD-1 pathway will bring about breakthroughs in the treatment of a variety of tumors: for the treatment of non-small cell lung cancer, renal cell carcinoma, ovarian cancer and melanoma (Homet M. B., Parisi G., et al., Anti-PD-1 Therapy in Melanoma. Semin Oncol., Jun. 2015, 42 (3): 466-473), and lymphoma and anemia (Held SA, Heine A, et al., Advances in immunotherapy of chronic myeloid leukemia CML. Curr Cancer Drug Targets., Sep. 2013, 13 (7): 768-74), microsatellite instability-high (MSI-H) or deficient mismatch repair (dMMR) tumors (several anti-PD-1 antibody drugs have been approved by the FDA et al for the treatment of tumors with MSI-H/dMMR characteristics). The current combination of anti-angiogenic therapy and immune checkpoint inhibitors shows good efficacy in many tumors. For example, the combination of the anti-VEGF antibodies (such as bevacizumab) and PD-1/PD-L1 antibodies (such as nivolumab, pembrolizumab and atezolizumab) for use in the treatment of ovarian cancer (Joyce F. Liu et al., JAMA Oncol., 2019; 5(12): 1731-1738), non-small cell lung cancer (non-small cell lung cancer including EGFR and/or ALK sensitive mutations) (Manegold C, et al., J Thorac Oncol., Feb. 2017; 12(2): 194-207), renal cell carcinoma (Dudek AZ et al., J Clin Oncol., 2018; 36 (suppl; abstr. 4558)), hepatocellular carcinoma (Stein S et al., J Clin Oncol, 2018, 36(15_suppl): 4074; bevacizumab in combination with atezolizumab for use in the treatment of hepatocellular carcinoma endorsed by the FDA in 2020), colorectal cancer (including MSI-H/dMMR and non-MSI-H/dMMR types) (Bendell JC et al., Safety and efficacy of MPDL3280A (anti-PDL1) in combination with bevacizumab (bev) and/or FOLFOX in patients (pts) with metastatic colorectal cancer (mCRC).

Presented at: American Society of Clinical Oncology; May 29-June 2, 2015; Chicago, IL. 2015; abstract 704; Hochster HS et al., Efficacy and safety of atezolizumab (atezo) and bevacizumab (bev) in a phase Ib study of microsatellite instability (MSI)-high metastatic colorectal cancer (mCRC). Presented at: American Society of Clinical Oncology Gastrointestinal Cancers Symposium; January 19-21, 2017; San Francisco, CA. 2017; abstract 673), breast cancer (Yukinori Ozaki et al., A multicenter phase II study evaluating the efficacy of nivolumab plus paclitaxel plus bevacizumab triple-combination therapy as a first-line treatment in patients with HER2-negative metastatic breast cancer: WJOG9917B NEWBEAT trial [abstract]. In: Proceedings of the 2019 San Antonio Breast Cancer Symposium; 2019 Dec 10-14; San Antonio, TX. Philadelphia (PA): AACR; Cancer Res 2020; 80(4 Suppl): Abstract nr PD1-03); the combination of VEGFR2 antibody and PD-1 antibody also showed good anti-tumor effects in adenocarcinoma of the stomach and gastroesophageal junction adenocarcinoma (Herbst RS et al., Lancet Oncol. 2019; 20(8): 1109-1123); also, the combination regimen of the PD-1 antibody (Pembrolizumab) and the angiogenesis inhibitor (Lenvatinib) showed good efficacy in the treatment of endometrial cancer, and was approved by the FDA in 2019 for use in the treatment of the endometrial cancer. For melanoma (PD-1 antibodies nivolumab and pembrolizumab have been approved by the FDA for use in the treatment of melanoma), cervical cancer (Krishnansu S. et al., N Engl J Med., 2014; 370: 734-743), glioma, prostate cancer (Antonarakis ES. et al., J Clin Oncol., Feb 10, 2020; 38(5): 395-405), urothelial cancer (Joaquim Bellmunt. et al., N Engl J Med., 2017; 376: 1015-1026; nivolumab for use in the treatment of bladder cancer endorsed by the FDA in 2017), esophageal cancer (Kato K et al., Lancet Oncol., 2019; 20(11): 1506-17), mesothelioma (Scherpereel A et al., Lancet Oncol., 2019; 20(2): 239-253) and the like, the PD-1/PDL-1 antibodies show good efficacy, and considering that the PD-1 pathway has a synergistic effect with the VEGF pathway in tumorigenesis, it can be expected that drugs that block both PD-1 and VEGF pathways will have better anti-tumor effects.

The bispecific antibody, also known as bispecific antibody, is a specific medicament that targets two different antigens simultaneously, and can be produced by immunoselection purification. In addition, the bispecific antibody can also be produced by genetic engineering, which has certain advantages due to corresponding flexibility in aspects such as the optimization of binding sites, consideration of synthetic form, and yield. Currently, the bispecific antibody has been demonstrated to exist in over 45 forms (Miller D, Kontermann RE. Bispecific antibodies for cancer immunotherapy: current perspectives. BioDrugs 2010; 24: 89-98). A number of bispecific antibodies have been developed in the form of IgG-ScFv, namely the Morrison form (Coloma M. J., Morrison S. L. Design and production of novel tetravalent bispecific antibodies. Nat Biotechnol., 1997; 15: 159-163), which has been demonstrated to be one of the ideal forms of the bispecific antibodies because of its similarity to the naturally existing IgG form and advantages in antibody engineering, expression and purification (Miller B. R., Demarest S. J., et al., Stability engineering of scFvs for the development of bispecific and multivalent antibodies. Protein Eng Des Sel 2010; 23: 549-57; Fitzgerald J, Lugovskoy A. Rational engineering of antibody therapeutics targeting multiple oncogene pathways. MAbs 2011; 3: 299-309).

ADCC (antibody-dependent cell-mediated cytotoxicity) refers to killing of a target cell by a killer cell (NK cells, macrophages, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (complement dependent cytotoxicity) refers to that the specific binding of an antibody to a corresponding antigen on a cell membrane surface forms a complex and activates the complement system, which further forms an MAC on the surface of the target cell resulting in subsequent target cell lysis. Complements may cause lysis of various bacteria and other pathogenic organisms, and are an important defense mechanism against pathogenic organism infections.

Fc receptors belong to an immunoglobulin family that are expressed on the surface of specific immune cells to recognize antibody Fc regions and mediate immune responses. After the Fab region recognizes an antigen, the Fc region of the antibody binds to the Fc receptor on the immune cell (e.g., a killer cell) to initiate the response function of the immune cell, such as phagocytosis and ADCC.

According to the type of antibody recognized by the Fc receptor and the type of expression cells, Fc receptors are mainly classified into three types, FcγR, FcαR and FcεR. FcγR can be further classified into four subtypes, FcγRI (CD64), FcγRII (CD32), FcγRIII (CD16) and FcRn (neonatal Fc receptor). Among these, FcγRI, FcγRII and FcγRIII are closely associated with ADCC effect.

FcγRIII is the most predominant molecule mediating ADCC, with two highly homologous subtypes, FcγRIIIa and FcγRIIIb, in different cell types. In FcγRIIIa populations, two subtypes distinguished by sites of single-nucleotide polymorphism (SNP), FcγRIIIa_V158 with high affinity and FcγRIIIa_F158 with low affinity, are present. FcγRI has higher affinity for the Fc region of IgG and participates in ADCC process; FcγRII comprises three subtypes of FcγRIIa, FcγRIIb and FcγRIIc (also referred to as CD32a, CD32b and CD32c respectively), among which FcγRIIa has ADCC activity; for FcγRIIa, two subtypes of FcγRIIa_H131 and FcγRIIa_R131 are present in humans due to single nucleotide mutation (Hogarth PM, Pietersz GA. 2012, NATURE REVIEWS DRUG DISCOVERY, 11(4): 311-331).

The IgG family comprises four members, IgG1, IgG2, IgG3 and IgG4, which differ in amino acids in the fragment crystallizable (Fc) region of the heavy chain constant region, resulting in their varying affinities for FcγRs. IgG1 is the most abundant subtype in humans and is also the most common subtype used in monoclonal antibody medication. IgG1 is capable of binding various FcγRs and is able to induce ADCC and CDC effects. IgG2 has the lowest affinity for FcγRs, but is still able to induce monocyte-mediated ADCC by binding to FcγRIIa. IgG3 features the highest binding capacity to FcγRs, and can induce ADCC and a greater CDC effect than IgG1. The IgG4 molecules bind weakly to FcγRs other than FcγRI, and the IgG4 molecules have a lower probability of causing CDC and NK cell-mediated ADCC. CN109053895A describes an anti-PD-1 and anti-VEGFA bifunctional antibody as well as a pharmaceutical composition and application thereof.

At present, there is still a need for developing a novel anti-PD-1-anti-VEGFA bispecific antibody to reduce or eliminate the damage caused by antibody-mediated ADCC and/or CDC activity against immune cells to which the anti-PD-1-anti-VEGFA bispecific antibody binds, and to improve the efficacy of the antibody drug.

SUMMARY

By intensive research and creative efforts, the inventor correspondingly modified the Fc fragment of the anti-PD-1-anti-VEGFA antibody structure to reduce the binding capacity of the Fc region to Fc receptors, thereby reducing ADCC and CDC toxic and side effects on immune cells and increasing the drug efficacy of the anti-PD-1-anti-VEGFA antibody drug.

The present invention is detailed below.

The present invention provides a bispecific antibody, comprising: (a) an immunoglobulin targeting VEGF-A, wherein the amino acid sequence of the heavy chain of the immunoglobulin is set forth in SEQ ID NO: 24, and the amino acid sequence of the light chain of the immunoglobulin is set forth in SEQ ID NO: 26; and (b) two scFvs targeting PD-1; wherein, for each of the two scFvs, the amino acid sequence of the heavy chain variable region is set forth in SEQ ID NO: 9, and the amino acid sequence of the light chain variable region is set forth in SEQ ID NO: 17; wherein: (i) one scFv is attached via a linker to the C-terminus of one of the two heavy chains of the immunoglobulin; and the other scFv is attached via a linker to the C-terminus of the other of the two heavy chains of the immunoglobulin; and (ii) for each of the two scFvs, the heavy chain variable region and the light chain variable region are attached via a linker; and wherein the linkers of (i) and the linkers of (ii) are the same or different, and are independently selected from SEQ ID NO: 18 and SEQ ID NO: 19. In an embodiment of the bispecific antibody of the invention, the amino acid sequences of the linkers of (i) and the amino acid sequences of the linkers of (ii) are set forth in SEQ ID NO: 18.

The bispecific antibody of the present invention may alternatively be described as a bispecific antibody comprising:

a first protein functional region targeting PD-1, and
a second protein functional region targeting VEGFA;
wherein:

the number of the first protein functional region is 2, and the number of the second protein functional region is 1;
the second protein functional region is an immunoglobulin, and the first protein functional region is a scFv;
the amino acid sequence of the heavy chain of the immunoglobulin is set forth in SEQ ID NO: 24, and the amino acid sequence of the light chain of the immunoglobulin is set forth in SEQ ID NO: 26;
the amino acid sequence of the heavy chain variable region of the scFv is set forth in SEQ ID NO: 9, and the amino acid sequence of the light chain variable region of the scFv is set forth in SEQ ID NO: 17;
the scFv is linked to the C-terminus of the heavy chain of the immunoglobulin;
the first protein functional region is linked to the second protein functional region via a first linker fragment;
and the heavy chain variable region of the scFv is linked to the light chain variable region of the scFv via a second linker fragment, and the first linker fragment and the second linker fragment are the same or different; and wherein the amino acid sequences of the first linker fragment and second linker fragment are independently selected from SEQ ID NO: 18 and SEQ ID NO: 19.

In a preferred embodiment, the amino acid sequences of the first linker fragment and second linker fragment are set forth in SEQ ID NO: 18.

The invention also provides an isolated nucleic acid molecule, encoding the bispecific antibody of the invention. The invention also provides a vector, comprising the isolated nucleic acid molecule according to the invention. The invention further provides a host cell, comprising the isolated nucleic acid molecule according to the invention or the vector according to the invention.

The invention further provides a conjugate, comprising the bispecific antibody according to the invention and a conjugated moiety, wherein the conjugated moiety is a detectable label; optionally wherein the conjugated moiety is a radioisotope, a fluorescent substance, a luminescent substance, a colored substance, or an enzyme.

The invention further provides a kit comprising the bispecific antibody according to the invention or the conjugate according to invention. Optionally, the kit further comprises a second antibody capable of specifically recognizing the immunoglobulin or an antigen binding fragment thereof. Further optionally, the second antibody further comprises a detectable label, for example, a radioisotope, a fluorescent substance, a luminescent substance, a colored substance, or an enzyme.

The invention further provides a pharmaceutical composition, comprising the bispecific antibody according to the invention. Optionally, the pharmaceutical composition further comprises a pharmaceutically acceptable vector and/or excipient.

The invention further provides the bispecific antibody according to the invention or the pharmaceutical composition according to the invention, for use in the treatment and/or prevention of a malignant tumor in a subject. In the medical uses of the invention, the malignant tumor to be treated and/or prevented with the antibody or composition of the invention may be selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia. The lung cancer may be non-small cell lung cancer or small cell lung cancer. The liver cancer may be hepatocellular carcinoma. The renal tumor may be renal cell carcinoma. The breast cancer may be triple negative breast cancer. The urothelial cancer may be bladder cancer.

In a preferred embodiment of the medical uses of the invention the non-small cell lung cancer is EGFR and/or ALK sensitive mutant non-small cell lung cancer. In a preferred embodiment of the medical uses of the invention the breast cancer is triple negative breast cancer.

In a preferred embodiment of the medical uses of the invention the bispecific antibody or pharmaceutical composition of the invention may be for administration by intravenous infusion or injection to the subject.

The invention further provides the bispecific antibody for use according to the invention or the pharmaceutical composition for use according to the invention, wherein the bispecific antibody or pharmaceutical composition is used in combination with a tumor chemotherapeutic drug, optionally wherein the tumor chemotherapeutic drug is administered prior to, concurrently with, or subsequent to the administration of the bispecific antibody or pharmaceutical composition.

In an embodiment, the bispecific antibody for use according to the invention or the pharmaceutical composition for use according to the invention is administered to the subject as a single administration, multiple administrations over a period of time, or, by reducing or increasing the dose proportionally with the emergency degree of the treatment.

In an embodiment of the medical uses of the invention, the bispecific antibody or pharmaceutical composition is administered to the subject at a dose of 0.02-50 mg/kg, 0.1-50 mg/kg, 0.1-25 mg/kg, or 1-10 mg/kg.

In one or more embodiments of the present invention, the bispecific antibody is in the form of IgG-scFv, i.e., the Morrison format.

The present disclosure provides a bispecific antibody wherein the heavy chain constant region of the immunoglobulin is human Ig gamma-1 chain C region or human Ig gamma-4 chain C region, and the light chain constant region of the immunoglobulin is human Ig kappa chain C region.

The present disclosure provides a bispecific antibody wherein the constant regions of the immunoglobulin are humanized. For example, the heavy chain constant region is Ig gamma-1 chain C region, ACCESSION: P01857, and the light chain constant region is Ig kappa chain C region, ACCESSION: P01834; or the heavy chain constant region of the immunoglobulin is Ig gamma-4 chain C region, ACCESSION: P01861.1; the light chain constant region of the immunoglobulin is Ig kappa chain C region, ACCESSION: P01834.

The present disclosure provides an amino acid sequence of the heavy chain constant region Ig gamma-1 chain C region (ACCESSION: P01857) as follows:

ASTKGPSVFPLAPSSKSTSGGTAALGCLVKDYFPEPVTVSWNSGALTSGVHTFPAVLQS

SGLYSLSSVVTVPSSSLGTQTYICNVNHKPSNTKVDKKVEPKSCDKTHTCPPCPAPELLG

GPSVFLFPPKPKDTLMISRTPEVTCVVVDVSHEDPEVKFNWYVDGVEVHNAKTKPREE

QYNSTYRVVSVLTVLHQDWLNGKEYKCKVSNKALPAPIEKTISKAKGQPREPQVYTLP

PSRDELTKNQVSLTCLVKGFYPSDIAVEWESNGQPENNYKTTPPVLDSDGSFFLYSKLT

VDKSRWQQGNVFSCSVMHEALHNHYTQKSLSLSPGK (SEQ ID NO: 40)

The present disclosure provides an amino acid sequence of the heavy chain constant region Ig gamma-4 chain C region (ACCESSION: P01861.1) as follows:

ASTKGPSVFPLAPCSRSTSESTAALGCLVKDYFPEPVTVSWNSGALTSGVHTFPAVLQSS

GLYSLSSVVTVPSSSLGTKTYTCNVDHKPSNTKVDKRVESKYGPPCPSCPAPEFLGGPS

VFLFPPKPKDTLMISRTPEVTCVVVDVSQEDPEVQFNWYVDGVEVHNAKTKPREEQFN

STYRVVSVLTVLHQDWLNGKEYKCKVSNKGLPSSIEKTISKAKGQPREPQVYTLPPSQE

EMTKNQVSLTCLVKGFYPSDIAVEWESNGQPENNYKTTPPVLDSDGSFFLYSRLTVDKS

RWQEGNVFSCSVMHEALHNHYTQKSLSLSLGK (SEQ ID NO: 41)

In one embodiment of the present invention, the amino acid sequence of the light chain constant region Ig kappa chain C region (ACCESSION: P01834) is as follows:

RTVAAPSVFIFPPSDEQLKSGTASVVCLLNNFYPREAKVQWKVDNALQSGNSQESVTE

QDSKDSTYSLSSTLTLSKADYEKHKVYACEVTHQGLSSPVTKSFNRGEC (SEQ ID NO:

42)

In some embodiments of the present invention, the bispecific antibody is provided, wherein the scFv is linked to the C-terminus of the heavy chain of the immunoglobulin. Since an immunoglobulin has two heavy chains, two scFvs are linked to one immunoglobulin molecule. Preferably, the two scFvs are identical.

In some embodiments of the present invention, the bispecific antibody is provided, wherein two scFvs are present, and one terminus of each scFv is linked to the C-terminus of one of the two heavy chains of the immunoglobulin.

In some embodiments of the present invention, a disulfide bond is present between the $V_H$ and the $V_L$ of the scFv. Methods for introducing a disulfide bond between the $V_H$ and $V_L$ of an antibody are well known in the art, see, for example, US 5,747,654; Rajagopal et. al., Prot. Engin., 10(1997)1453-1459; Reiter et. Al., Nat. Biotechnol., 14(1996)1239-1245; Reiter et. al., Protein Engineering, 8(1995)1323-1331; Webber et. al., Molecular Immunology, 32(1995)249-258; Reiter et. al., Immunity, 2(1995)281-287; Reiter et. al., JBC, 269(1994)18327-18331; Reiter et. al., Inter. J. of Cancer, 58(1994) 142-149; or Reiter et al., Cancer Res. 54(1994)2714-2718. Preferably, the amino acid sequences of the first linker fragment and second linker fragments are set forth in SEQ ID NO: 18.

In one or more embodiments of the present invention, the bispecific antibody is provided, wherein the immunoglobulin or an antigen-binding fragment thereof binds to FcγRI with an affinity constant of less than about $10^{-6}$ M, such as less than about $10^{-7}$ M, $10^{-8}$ M or $10^{-9}$ M or less; preferably, the affinity constant is measured by a Fortebio Octet system.

In one or more embodiments of the present invention, the bispecific antibody is provided, wherein the immunoglobulin or the antigen-binding fragment thereof binds to C1q with an affinity constant of less than about $10^{-9}$ M, such as less than about $10^{-7}$ M, $10^{-8}$ M or $10^{-9}$ M or less; preferably, the affinity constant is measured by a Fortebio Octet system.

In some embodiments of the present invention, the bispecific antibody is provided, wherein the bispecific antibody binds to a VEGFA protein and/or a PD-1 protein with a $K_D$ of less than $10^{-5}$ M, such as less than $10^{-6}$ M, $10^{-7}$ M, $10^{-8}$ M, $10^{-9}$ M or $10^{-10}$ M or less; preferably, the $K_D$ is measured by a Fortebio system.

In some embodiments of the present invention, the bispecific antibody is provided, wherein,

the bispecific antibody binds to the VEGFA protein with an $EC_{50}$ of less than 1 nM, less than 0.5 nM, less than 0.2 nM, less than 0.15 nM, or less than 0.14 nM; preferably, the $EC_{50}$ is detected by indirect ELISA; and/or,
the bispecific antibody binds to the PD-1 protein with an $EC_{50}$ of less than 1 nM, less than 0.5 nM, less than 0.2 nM, less than 0.17 nM, less than 0.16 nM, or less than 0.15 nM; preferably, the $EC_{50}$ is detected by indirect ELISA.

In one or more embodiments of the present invention, the bispecific antibody is a monoclonal antibody.

In one or more embodiments of the present invention, the bispecific antibody is a humanized antibody.

Another aspect of the present invention relates to an isolated nucleic acid molecule encoding the bispecific antibody according to any embodiment of the present invention.

Yet another aspect of the present invention relates to a vector comprising the isolated nucleic acid molecule disclosed herein.

Yet another aspect of the present invention relates to a host cell comprising the isolated nucleic acid molecule or the vector described herein.

Another aspect of the present invention relates to a conjugate comprising an antibody or an antigen-binding fragment thereof and a conjugated moiety, wherein the immunoglobulin is the bispecific antibody according to any embodiment of the present invention, and the conjugated moiety is a detectable label; preferably, the conjugated moiety is a radioisotope, a fluorescent substance, a luminescent substance, a colored substance, or an enzyme.

Another aspect of the present invention relates to a kit comprising the bispecific antibody according to any embodiment of the present invention or comprising the conjugate of the present invention; wherein optionally, the kit further comprises a second antibody capable of specifically recognizing the immunoglobulin or the antigen binding fragment thereof; optionally, the second antibody further comprises a detectable label, for example, a radioisotope, a fluorescent substance, a luminescent substance, a colored substance, or an enzyme.

The present disclosure provides a bispecific antibody or the conjugate according to any embodiment of the present invention which may be used in preparing a kit for detecting the presence or level of PD-1 and/or VEGFA in a sample.

Another aspect of the present invention relates to a pharmaceutical composition comprising the bispecific antibody or the conjugate according to any embodiment of the present invention; optionally, the pharmaceutical composition further comprises a pharmaceutically acceptable vector and/or excipient.

The bispecific antibody of the present invention or the pharmaceutical composition of the present invention may be formulated into any dosage form known in the pharmaceutical field, such as tablet, pill, suspension, emulsion, solution, gel, capsule, powder, granule, elixir, troche, suppository, injection (including injection solution, sterile powder for injection and concentrated solution for injection), inhalant, and spray. The preferred dosage form depends on the intended mode of administration and therapeutic use. The pharmaceutical composition of the present invention should be sterile and stable under the conditions of manufacture and storage. One preferred dosage form is an injection. Such injections may be sterile injection solutions. For example, sterile injection solutions can be prepared by the following method: a necessary amount of the bispecific antibody of the present invention is added in an appropriate solvent, and optionally, other desired ingredients (including, but not limited to, pH regulators, surfactants, adjuvants, ionic strength enhancers, isotonic agents, preservatives, diluents, or any combination thereof) are added at the same time, followed by filtration and sterilization. In addition, sterile injection solutions can be prepared as sterile lyophilized powders (e.g., by vacuum drying or lyophilizing) for convenient storage and use. Such sterile lyophilized powders may be dispersed in a suitable vector (e.g., sterile pyrogen-free water) prior to use.

In addition, the bispecific antibody of the present invention may be present in a pharmaceutical composition in unit dose form for ease of administration. In some embodiments, the unit dose is at least 1 mg, at least 5 mg, at least 10 mg, at least 15 mg, at least 20 mg, at least 25 mg, at least 30 mg, at least 45 mg, at least 50 mg, at least 75 mg, or at least 100 mg. Where the pharmaceutical composition is in a liquid (e.g., injection) dosage form, it may comprise the bispecific antibody of the present invention at a concentration of at least 0.1 mg/mL, such as at least 0.25 mg/mL, at least 0.5 mg/mL, at least 1 mg/mL, at least 2.5 mg/mL, at least 5 mg/mL, at least 8 mg/mL, at least 10 mg/mL, at least 15 mg/mL, at least 25 mg/mL, at least 50 mg/mL, at least 75 mg/mL, or at least 100 mg/mL.

The bispecific antibody or the pharmaceutical composition of the present invention may be administered by any suitable method known in the art, including, but not limited to, oral, buccal, sublingual, ocular, topical, parenteral, rectal, intrathecal, intracisternal, inguinal, intravesical, topical (e.g., powder, ointment, or drop), or nasal route. However, for many therapeutic uses, the preferred route/mode of administration is parenteral (such as intravenous injection, subcutaneous injection, intraperitoneal injection, and intramuscular injection). Those skilled in the art will appreciate that the route and/or mode of administration will vary depending on the intended purpose. In a preferred embodiment, the bispecific antibody or the pharmaceutical composition of the present invention is administered by intravenous infusion or injection.

The bispecific antibody or the pharmaceutical composition provided herein can be used alone or in combination, or used in combination with additional pharmaceutically active agents (e.g., a tumor chemotherapeutic drug). Such an additional pharmaceutically active agent may be administered prior to, concurrently with, or subsequent to the administration of the bispecific antibody of the present invention or the pharmaceutical composition of the present invention.

In the present invention, the administration regimen may be adjusted to achieve the optimal desired response (e.g., a therapeutic or prophylactic response). For example, it may be a single administration, may be multiple administrations over a period of time, or may be characterized by reducing or increasing the dose proportionally with the emergency degree of the treatment.

A further aspect of the present invention relates to the bispecific antibody or the conjugate as described herein for use in the treatment and/or prevention of a malignant tumor; preferably, the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia;

preferably, the lung cancer is non-small cell lung cancer or small cell lung cancer; preferably, the non-small cell lung cancer is EGFR and/or ALK sensitive mutant non-small cell lung cancer;
preferably, the liver cancer is hepatocellular carcinoma;
preferably, the renal tumor is renal cell carcinoma;
preferably, the breast cancer is triple negative breast cancer;
preferably, the urothelial cancer is bladder cancer.

The present disclosure also contemplates that the bispecific antibody or the conjugate may be used for:

(1)

detecting the level of VEGFA in a sample,
blocking the binding of VEGFA to VEGFR2,

down-regulating the activity or level of VEGFA,
relieving the stimulation of VEGFA on vascular endothelial cell proliferation,
inhibiting vascular endothelial cell proliferation, or
blocking tumor angiogenesis;

and/or
(2)

blocking the binding of PD-1 to PD-L1,
down-regulating the activity or level of PD-1,
relieving the immunosuppression of PD-1 in an organism,
promoting IFN-y secretion in T lymphocytes, or
promoting IL-2 secretion in T lymphocytes.

Antibody drugs, especially monoclonal antibodies, have achieved good efficacy in the treatment of various diseases. Traditional experimental methods for acquiring these therapeutic antibodies are to immunize animals with the antigen and acquire antibodies targeting the antigen in the immunized animals, or to improve those antibodies with lower affinity for the antigen by affinity maturation.

The variable regions of the light chain and the heavy chain determine the binding of the antigen; the variable region of each chain contains three hypervariable regions called complementarity determining regions (CDRs). CDRs of the heavy chain (H Chain) comprise HCDR1, HCDR2, and HCDR3, and CDRs of the light chain (L Chain) comprise LCDR1, LCDR2, and LCDR3, which are named by Kabat et al., see Bethesda M.d., Sequences of Proteins of Immunological Interest, Fifth Edition, NIH Publication 1991, (1-3): 91-3242.

Preferably, CDRs may also be defined by the IMGT numbering system, see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 38.suppl_1 (2009): D301-D307. The amino acid sequences of the CDR regions of the monoclonal antibody sequences in (1) to (13) below are analyzed by technical means well known to those skilled in the art, for example by VBASE2 database and according to the IMGT definition, and the results are as follows:

(1) Bevacizumab

The amino acid sequence of the heavy chain variable region is set forth in SEQ ID NO: 1, and the amino acid sequence of the light chain variable region is set forth in SEQ ID NO: 3.

The amino acid sequences of the 3 CDR regions of the heavy chain variable region are as follows:

HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)

the amino acid sequences of the 3 CDR regions of the light chain variable region are as follows:

LCDR1: QDISNY (SEQ ID NO: 31)
LCDR2: FTS (SEQ ID NO: 32)
LCDR3: QQYSTVPWT (SEQ ID NO: 33)

(2) 14C12, 14C12H1L1 or 14C12H1L1 (M)

The amino acid sequences of the 3 CDR regions of the heavy chain variable region are as follows:

HCDR1: GFAFSSYD (SEQ ID NO: 34)
HCDR2: ISGGGRYT (SEQ ID NO: 35)
HCDR3: ANRYGEAWFAY (SEQ ID NO: 36)

the amino acid sequences of the 3 CDR regions of the light chain variable region are as follows:

LCDR1: QDINTY (SEQ ID NO: 37)
LCDR2: RAN (SEQ ID NO: 38)

LCDR3: LQYDEFPLT (SEQ ID NO: 39)

(3) <u>VP101 (hG1WT) or VP101 (hG1DM)</u>

The amino acid sequences of the 9 CDR regions of its heavy chains are as follows:

HCDR1: GYTFTNYG (SEQ ID NO: 28)
HCDR2: INTYTGEP (SEQ ID NO: 29)
HCDR3: AKYPHYYGSSHWYFDV (SEQ ID NO: 30)
HCDR4: GFAFSSYD (SEQ ID NO: 34)
HCDR5: ISGGGRYT (SEQ ID NO: 35)
HCDR6: ANRYGEAWFAY (SEQ ID NO: 36)
HCDR7: QDINTY (SEQ ID NO: 37)
HCDR8: RAN (SEQ ID NO: 38)
HCDR9: LQYDEFPLT (SEQ ID NO: 39)

the amino acid sequences of the 3 CDR regions of the light chain variable region are as follows:

LCDR1: QDISNY (SEQ ID NO: 31)
LCDR2: FTS (SEQ ID NO: 32)
LCDR3: QQYSTVPWT (SEQ ID NO: 33).

For the antibody VP101 (hG1DM) of the present invention, amino acid mutations are introduced into the non-variable region of VP101 (hG1WT). According to the EU numbering system, amino acid mutations are introduced at positions 234 and 235:
VP101 (hG1DM) is obtained by introducing a leucine-to-alanine point mutation at position 234 (L234A) and a leucine-to-alanine point mutation at position 235 (L235A) in the hinge region of the heavy chain.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, when referring to the amino acid sequence of VEGFA protein (GenBank ID: NP_001165097.1), it includes the full length of the VEGFA protein, as well as a fusion protein of VEGFA, such as a fragment fused to an Fc protein fragment of mouse or human IgG (mFc or hFc). However, those skilled in the art will appreciate that in the amino acid sequence of the VEGFA protein, mutations or variations (including but not limited to, substitutions, deletions and/or additions) can be naturally generated or artificially introduced without affecting biological functions thereof. Therefore, in the present invention, the term "VEGFA protein" should include all such sequences, including their natural or artificial variants. In addition, when describing the sequence fragment of the VEGFA protein, it also includes the corresponding sequence fragments in its natural or artificial variants. In one embodiment of the present invention, the amino acid sequence of the VEGFA protein is shown as the underlined part of SEQ ID NO: 33 (without the last 6 His, a total of 302 amino acids).

As used herein, when referring to the amino acid sequence of VEGFR2 protein (also known as KDR, GenBank ID: NP_002244), it includes the full length of the VEGFR2 protein, or the extracellular fragment VEGFR2-ECD of VEGFR2, or a fragment comprising VEGFR2-ECD, and it also includes a fusion protein of VEGFR2-ECD, such as a fragment fused to an Fc protein fragment of mouse or human IgG (mFc or hFc). However, those skilled in the art will appreciate that in the amino acid sequence of the VEGFR2 protein, mutations or variations (including but not limited to, substitutions, deletions and/or additions) can be naturally generated or artificially introduced without affecting biological functions thereof. Therefore, in the present invention, the term "VEGFR2 protein" should include all such sequences, including their natural or artificial variants. In addition, when describing the sequence fragment of the VEGFR2 protein, it also includes the corresponding sequence fragments in its natural or artificial variants. In one embodiment of the present invention, the amino acid sequence of the extracellular fragment VEGFR2-ECD of VEGFR2 is set forth in SEQ ID NO: 34 (766 amino acids).

As used herein, unless otherwise specified, the VEGFR is VEGFR1 and/or VEGFR2; specific protein sequence thereof is a sequence known in the prior art, and reference may be made to the sequence disclosed in the existing literature or GenBank. For example, VEGFR1 (VEGFR1, NCBI Gene ID: 2321); VEGFR2 (VEGFR2, NCBI Gene ID: 3791).

As used herein, when referring to the amino acid sequence of PD-1 protein (programmed cell death protein 1, NCBI GenBank: NM_005018), it includes the full length of the PD-1 protein, or the extracellular fragment PD-1ECD of PD-1 or a fragment comprising PD-1ECD, and it further includes a fusion protein of PD-1ECD, such as a fragment fused to an Fc protein fragment of a mouse or human IgG (mFc or hFc). However, it will be appreciated by those skilled in the art that in the amino acid sequence of PD-1 protein, mutations or variations (including but not limited to substitutions, deletions and/or additions) can be naturally generated or artificially introduced without affecting biological functions thereof. Therefore, in the present invention, the term "PD-1 protein" should include all such sequences, including their natural or artificial variants. In addition, when describing the sequence fragment of the PD-1 protein, it also includes the corresponding sequence fragments in its natural or artificial variants.

As used herein, the term $EC_{50}$ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). In a general sense, the heavy chain can be interpreted as a polypeptide chain with a larger molecular weight in an antibody, and the light chain refers to a polypeptide chain with a smaller molecular weight in an antibody. Light chains are classified as $\kappa$ and $\lambda$ light chains. Heavy chains are generally classified as $\mu$, $\delta$, $\gamma$, $\alpha$, or $\varepsilon$, and isotypes of antibodies are defined as IgM, IgD, IgG, IgA, and IgE, respectively. In light chains and heavy chains, the variable region and constant region are linked by a "J" region of about 12 or more amino acids, and the heavy chain also comprises a "D" region of about 3 or more amino acids. Each heavy chain consists of a heavy chain variable region ($V_H$) and a heavy chain constant region ($C_H$). The heavy chain constant region consists of 3 domains ($C_{H1}$, $C_{H2}$, and $C_{H3}$). Each light chain consists of a light chain variable region ($V_L$) and a light chain constant region ($C_L$). The light chain constant region consists of one domain $C_L$. The constant region of the antibody can mediate the binding of immunoglobulins to host tissues or factors, including the binding of various cells of the immune system (e.g., effector cells) to the first component (C1q) of classical complement system. The $V_H$ and $V_L$ regions can be further subdivided into highly variable regions (called complementarity determining regions (CDRs)), between which conservative regions called framework regions (FRs) are distributed. Each $V_H$ and $V_L$ consists of 3 CDRs and 4 FRs arranged from amino terminus to carboxyl terminus in the following order: FR1, CDR1, FR2, CDR2, FR3, CDR3, FR4. The variable regions ($V_H$ and $V_L$) of each heavy chain/light chain pair form an antibody binding site. The assignment of amino acids to the regions or domains may be based on Kabat Sequences of Proteins of Immunological Interest (National Institutes of Health, Bethesda, Md. (1987 and 1991)), or Chothia & Lesk J. Mol. Biol. 196(1987): 901-917; Chothia et al. Nature 342(1989): 878-883 or the definition of IMGT numbering system, see Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 38.suppl_1 (2009): D301-D307. In particular, the heavy chain may also comprise more than 3 CDRs, such as 6, 9, or 12.

The term "antibody" is not limited by any specific method for producing the antibody. As used herein, the term "antigen binding fragment", also known as the "antigen binding portion", refers to a polypeptide comprising the fragment of a full-length antibody, which maintains the ability to specifically bind to the same antigen to which the full-length antibody binds, and/or competes with the full-length antibody for the specific binding to an antigen. See generally, Fundamental Immunology, Ch. 7 (Paul, W., ed., 2nd edition, Raven Press, N.Y. (1989). An antigen-binding fragment of an antibody can be produced by recombinant DNA techniques or by enzymatic or chemical cleavage of an intact antibody.

As used herein, the term "Fd fragment" refers to an antibody fragment consisting of $V_H$ and $C_{H1}$ domains; the term "Fv fragment" refers to an antibody fragment consisting of the $V_L$ and $V_H$ domains of a single arm of an antibody; the term "dAb fragment" refers to an antibody fragment consisting of a $V_H$ domain (Ward et al., Nature 341 (1989): 544-546); the term "Fab fragment" refers to an antibody fragment consisting of $V_L$, $V_H$, $C_L$ and $C_{H1}$ domains; and the term "F(ab')$_2$ fragment" refers to an antibody fragment comprising two Fab fragments linked by the disulfide bridge on a hinge region.

In some cases, the antigen binding fragment of the antibody is a single chain antibody (e.g., scFv) in which the $V_L$ and $V_H$ domains are paired to form a monovalent molecule via a linker that enables them to produce a single polypeptide chain (see, e.g., Bird et al., Science 242 (1988): 423-426 and Huston et al., Proc. Natl. Acad. Sci. USA 85 (1988): 5879-5883). Such scFv molecules may have a general structure: $NH_2$-$V_L$-linker-$V_H$-COOH or $NH_2$-$V_H$-linker-$V_L$-COOH. An appropriate linker in prior art consists of a repeating GGGGS amino acid sequence or a variant thereof. For example, a linker having the amino acid sequence (GGGGS)$_4$ can be used, and variants thereof can also be used (Holliger et al., Proc. Natl. Acad. Sci. USA 90 (1993): 6444-6448). Other linkers useful in the present invention are described by Alfthan et al., Protein Eng. 8 (1995): 725-731, Choi et al., Eur. J. Immunol., 31 (2001): 94-106, Hu et al., Cancer Res. 56 (1996): 3055-3061, Kipriyanov et al., J. Mol. Biol., 293 (1999): 41-56, and Roovers et al., Cancer Immunol. (2001).

In some cases, the antigen binding fragment of the antibody is a diabody, that is, a bivalent antibody, in which the $V_H$ and $V_L$ domains are expressed on a single polypeptide chain. However, the linker used is too short to allow the pairing of the two domains on the same chain, thereby the domains are forced to pair with the complementary domains on the other chain and two antigen binding sites are generated (see, e.g., Holliger P. et al., Proc. Natl. Acad. Sci. USA 90 (1993): 6444-6448, and Poljak R. J. et al., Structure 2 (1994): 1121-1123).

Antigen binding fragments (e.g., the above mentioned antibody fragments) of antibodies can be obtained from given antibodies by using conventional techniques known to those skilled in the art (e.g., recombinant DNA technique, or enzymatic or chemical cleavage), and the antigen binding fragments of the antibodies are screened for specificity in the same way as for intact antibodies.

As used herein, unless otherwise clearly defined in the context, when referring to the term "antibody", it includes not only intact antibodies but also antigen binding fragments of antibodies. As used herein, the terms "mAb" and "monoclonal antibody" refer to an antibody or a fragment thereof that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained by hybridoma technique first reported by Kohler et al. (Nature, 256: 495, 1975), and can also be obtained by recombinant DNA technique (for example, see U.S. Patent No. 4,816,567).

As used herein, the term "chimeric antibody" refers to an antibody of which a part of the light or/and heavy chains is derived from an antibody (which may be derived from a specific species or belong to a specific antibody class or subclass), and the other part of the light or/and heavy chains are derived from another antibody (which may be derived from the same or different species or belong to the same or different antibody class or subclass). But in any case, it retains the binding activity for the target antigen (U.S. Patent 4,816,567, Cabilly et al.; Morrison et al., Proc. Natl. Acad. Sci. USA, 81 (1984): 6851-6855).

As used herein, the term "humanized antibody" refers to an antibody or antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986, 321: 522-525; Reichmann et al., Nature, 1988, 332: 323 329; Presta, Curr. Op. Struct. Biol., 1992, 2: 593-596, and Clark, Immunol. Today 2000, 21: 397-402.

As used herein, the term "epitope" refers to a site on the antigen that an immunoglobulin or antibody specifically binds to. "Epitope" is also referred to in the field as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally comprises at least 3, 4, 5, 6, 7, 8, 9, 10, 11, 12, 13, 14, or 15 consecutive or non-consecutive amino acids in a unique spatial conformation, which can be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

As used herein, the term "isolated" refers to obtaining by artificial means from natural state. If a certain "isolated" substance or component appears in nature, it may be the case that change occurs in its natural environment, or that it is isolated from the natural environment, or both. For example, a certain non-isolated polynucleotide or polypeptide naturally occurs in a certain living animal, and the same polynucleotide or polypeptide with high purity isolated in such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows for the expression of the protein encoded by the inserted polynucleotide, the vector is referred to as an expression vector. The vector can be introduced into a host cell by transformation, transduction, or transfection so that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as E. coli or bacillus subtilis, fungal cells such as yeast cells or aspergillus, insect cells such as S2 drosophila cells or Sf9, or animal cells such as fibroblasts, CHO cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) refers to that the antibody binds to the antigen with an affinity ($K_D$) of less than about $10^{-5}$ M, such as less than about $10^{-6}$ M, $10^{-7}$ M, $10^{-8}$ M, $10^{-9}$ M or $10^{-10}$ M or less. In some embodiments of the present invention, the term "target" refers to specific binding.

As used herein, the term "$K_D$" refers to a dissociation equilibrium constant for a specific antibody-antigen interaction, which is used to describe the binding affinity between the antibody and the antigen. A smaller equilibrium dissociation constant indicates a stronger antibody-antigen binding and a higher affinity between the antibody and the antigen. Generally, an antibody binds to an antigen with a dissociation equilibrium constant ($K_D$) of less than about $10^{-5}$ M, such as less than about $10^{-6}$ M, $10^{-7}$ M, $10^{-8}$ M, $10^{-9}$ M or $10^{-10}$ M or less, for example, as determined on a BIACORE surface plasmon resonance (SPR) instrument or a Fortebio system.

As used herein, the terms "monoclonal antibody" and "mAb" have the same meaning and can be used interchangeably; the terms "polyclonal antibody" and "pAb" have the same meaning and can be used interchangeably; the terms "polypeptide" and "protein" have the same meaning and can be used interchangeably. Besides, herein, amino acids are generally represented by singleletter and three-letter abbreviations known in the art. For example, alanine can be represented by A or Ala.

As used herein, the term "pharmaceutically acceptable auxiliary material" refers to a vector and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995) and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "adjuvant" refers to a non-specific immune enhancer, which can enhance the immune response of an organism to antigens or change the type of immune response when delivered into the organism together with the antigens or in advance. There are various adjuvants, including, but not limited to, aluminum adjuvant (e.g., aluminum hydroxide), Freund's adjuvant (e.g., complete Freund's adjuvant and incomplete Freund's adjuvant), *Corynebacterium parvum,* lipopolysaccharide, cytokine, etc. The Freund's adjuvant is the most commonly used adjuvant in animal experiments. The aluminum hydroxide adjuvant is used more frequently in clinical trials. As used herein, the term "effective amount" refers to an amount sufficient to obtain or at least partially obtain desired effects. For example, a prophylactically effective amount (e.g., for a disease associated with PD-1 binding to PD-L1 or overexpression of VEGF, such as a tumor) is an amount sufficient to prevent, stop, or delay the onset of the disease (e.g., a disease associated with PD-1 binding to PD-L1 or overexpression of VEGF, such as a tumor); a therapeutically effective amount is an amount sufficient to cure or at least partially stop the disease and its complications in a patient suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

The term "MSI" refers to microsatellite instability. Microsatellites are short tandem repeats throughout the human genome, including 10-50 repeats of one, two or more nucleotides. Microsatellites in certain abnormal cells, such as tumors, are altered in length by insertion or deletion of repeat units as compared to normal cells. Such alteration is referred to as MSI. Based on instability and extent, MSI can be classified as microsatellite instability-high (MSI-H), microsatellite instability-low (MSI-L) and microsatellite stable (MSS). The major cause of MSI is DNA mismatch repair (MMR) deficiency. Human mismatch repair genes (MMR genes) can express corresponding mismatch repair proteins through transcription and translation. Absence of any MMR protein may lead to mismatch repair deficiency, and basepair mismatch will accumulate in the process of DNA replication due to such deficiency, ultimately resulting in MSI. About 15% of colorectal cancers are attributed to the MSI pathway. This was first reported in colorectal cancer, and may also occur in gastric cancer, endometrial cancer, adrenocortical carcinoma and the like (Baretti M et al., Pharmacol Ther., 2018; 189: 45-62). MSI-H/dMMR characteristics were also found in mesothelioma, sarcoma, adrenocortical carcinoma, malignant melanoma and ovarian germ cell neoplasm in subsequent studies.

MSI-H and dMMR represent the results of two different assays and are biologically consistent, called MSI-H/dMMR or MSI-high/dMMR, while MSI-L and MSS are phenotypes of proficient mismatch repair (pMMR). The detection of dMMR is to perform an immunohistochemical assay of protein expression for four mismatch genes of MSH2, MLH1, MSH6 and PMS2 based on tumor specimens (including surgical specimens and aspiration specimens). Absence of any of the four proteins confirms the dMMR; positive results of all the four proteins indicate pMMR, i.e., a complete mismatch repair function. The detection of MSI is to match the length of the repeated DNA sequences (microsatellite sequences) in tumor cells and somatic cells, and to compare the lengths. When 5 standard loci are detected using PCR based on the American NCI standard, inconsistencies in two or more loci indicate instability, defined as MSI-H, one inconsistent locus indicates MSI-L, and 5 consistent loci indicate MSS. High-throughput sequencing (also referred to as next-generation sequencing, or NGS) can also be used as a method for detecting microsatellite instability. When more microsatellite loci are selected, such as more than 5 loci or additional microsatellite loci, for PCR assay, inconsistency in ≥30% loci is defined as MSI-H, consistency in all loci is defined as MSS, and inconsistency between 0 and 30% is defined as MSI-L.

Beneficial Effects

The present invention achieves one or more of the following technical effects (1) to (4):

(1) The modification of the Fc fragment of the antibody of the present invention completely eliminates the binding activity of VP101(hG1WT) and $F_C$ receptors FcγRI and FcγRIIIa_F158, thereby completely eliminating the ADCC activity.
(2) The modification of the Fc fragment of the antibody of the present invention completely eliminates the binding activity of VP101(hG1WT) and the complement C1q, thereby completely eliminating the CDC activity.
(3) The bispecific antibody of the present invention can specifically bind to VEGFA well, can effectively block the binding of VEGFA to VEGFR2, and specifically relieves the immunosuppression of VEGFA in an organism and the promotion effect of VEGFA on angiogenesis.
(4) The bispecific antibody of the present invention can specifically bind to PD-1 well, can effectively block the binding of PD-1 to PD-L1, and specifically relieves the immunosuppression of PD-1 in an organism and activate the immune response.

BRIEF DESCRIPTION OF THE DRAWINGS

FIG. 1: Affinity constant assay of VP101(hG1DM) to FcγRI. The antibody concentrations for the curve pairs from top to bottom are 50 nM, 25 nM, 12.5 nM, 6.25 nM and 3.12 nM, respectively.
FIG. 2: Affinity constant assay of bevacizumab to FcγRI. The antibody concentrations for the curve pairs from top to bottom are 50 nM, 25 nM, 12.5 nM, 6.25 nM and 3.12 nM, respectively.
FIG. 3: Affinity constant assay of nivolumab to FcγRI. The antibody concentrations for the curve pairs from top to bottom are 50 nM, 25 nM, 12.5 nM, 6.25 nM and 3.12 nM, respectively.
FIG. 4: Affinity constant assay of VP101(hG1WT) to FcγRI. The antibody concentrations for the curve pairs from top to bottom are 50 nM, 25 nM, 12.5 nM, 6.25 nM and 3.12 nM, respectively.
FIG. 5: Affinity constant assay of VP101(hG4WT) to FcγRI. The antibody concentrations for the curve pairs from top to bottom are 50 nM, 25 nM, 12.5 nM, 6.25 nM and 3.12 nM, respectively.
FIG. 6: Affinity constant assay of VP101(hG1DM) to FcγRIIa_H131. The antibody concentrations for the curve pairs from top to bottom are 200 nM, 100 nM, 50 nM, 25 nM and 12.5 nM, respectively.
FIG. 7: Affinity constant assay of bevacizumab to FcγRIIa_H131. The antibody concentrations for the curve pairs from top to bottom are 200 nM, 100 nM, 50 nM, 25 nM and 12.5 nM, respectively.
FIG. 8: Affinity constant assay of nivolumab to FcγRIIa_H131. The antibody concentrations for the curve pairs from top to bottom are 200 nM, 100 nM, 50 nM, 25 nM and 12.5 nM, respectively.
FIG. 9: Affinity constant assay of VP101(hG1WT) to FcγRIIa_H131. The antibody concentrations for the curve pairs from top to bottom are 200 nM, 100 nM, 50 nM, 25 nM and 12.5 nM, respectively.
FIG. 10: Affinity constant assay of VP101(hG4WT) to FcγRIIa_H131. The antibody concentrations for the curve pairs from top to bottom are 200 nM, 100 nM, 50 nM, 25 nM and 12.5 nM, respectively.
FIG. 11: Affinity constant assay of VP101(hG1DM) to FcγRIIa_R131. The antibody concentrations for the curve pairs from top to bottom are 200 nM, 100 nM, 50 nM, 25 nM and 12.5 nM, respectively.
FIG. 12: Affinity constant assay of bevacizumab to FcγRIIa_R131. The antibody concentrations for the curve pairs from top to bottom are 200 nM, 100 nM, 50 nM, 25 nM and 12.5 nM, respectively.
FIG. 13: Affinity constant assay of nivolumab to FcγRIIa_R131. The antibody concentrations for the curve pairs from top to bottom are 200 nM, 100 nM, 50 nM, 25 nM and 12.5 nM, respectively.
FIG. 14: Affinity constant assay of VP101(hG1WT) to FcγRIIa_R131. The antibody concentrations for the curve pairs from top to bottom are 200 nM, 100 nM, 50 nM, 25 nM and 12.5 nM, respectively.
FIG. 15: Affinity constant assay of VP101(hG4WT) to FcγRIIa_R131. The antibody concentrations for the curve pairs from top to bottom are 200 nM, 100 nM, 50 nM, 25 nM and 12.5 nM, respectively.
FIG. 16: Affinity constant assay of VP101(hG1DM) to FcγRIIIa_V158. The antibody concentrations for the curve pairs from top to bottom are 500 nM, 250 nM, 125 nM, 62.5 nM and 31.25 nM, respectively.
FIG. 17: Affinity constant assay of bevacizumab to FcγRIIIa_V158. The antibody concentrations for the curve pairs from top to bottom are 500 nM, 250 nM, 125 nM, 62.5 nM and 31.25 nM, respectively.

FIG. 18: Affinity constant assay of nivolumab to Fc$\gamma$RIIIa_V158. The antibody concentrations for the curve pairs from top to bottom are 500 nM, 250 nM, 125 nM, 62.5 nM and 31.25 nM, respectively.

FIG. 19: Affinity constant assay of VP101(hG1WT) to Fc$\gamma$RIIIa_V158. The antibody concentrations for the curve pairs from top to bottom are 500 nM, 250 nM, 125 nM, 62.5 nM and 31.25 nM, respectively.

FIG. 20: Affinity constant assay of VP101(hG4WT) to Fc$\gamma$RIIIa_V158. The antibody concentrations for the curve pairs from top to bottom are 500 nM, 250 nM, 125 nM, 62.5 nM and 31.25 nM, respectively.

FIG. 21: Affinity constant assay of VP101(hG1DM) to Fc$\gamma$RIIIa_F158. The antigen concentrations for the curve pairs from top to bottom are 500 nM, 250 nM, 125 nM, 62.5 nM and 31.25 nM, respectively.

FIG. 22: Affinity constant assay of bevacizumab to Fc$\gamma$RIIIa_F158. The antibody concentrations for the curve pairs from top to bottom are 500 nM, 250 nM, 125 nM, 62.5 nM and 31.25 nM, respectively.

FIG. 23: Affinity constant assay of nivolumab to Fc$\gamma$RIIIa_F158. The antibody concentrations for the curve pairs from top to bottom are 500 nM, 250 nM, 125 nM, 62.5 nM and 31.25 nM, respectively.

FIG. 24: Affinity constant assay of VP101(hG1WT) to Fc$\gamma$RIIIa_F158. The antibody concentrations for the curve pairs from top to bottom are 500 nM, 250 nM, 125 nM, 62.5 nM and 31.25 nM, respectively.

FIG. 25: Affinity constant assay of VP101(hG4WT) to Fc$\gamma$RIIa_F158. The antibody concentrations for the curve pairs from top to bottom are 500 nM, 250 nM, 125 nM, 62.5 nM and 31.25 nM, respectively.

FIG. 26: Affinity constant assay of VP101(hG1DM) to C1q. The antibody concentrations for the curve pairs from top to bottom are 10 nM, 5 nM, 2.5 nM, 1.25 nM and 0.625 nM, respectively.

FIG. 27: Affinity constant assay of bevacizumab to C1q. The antibody concentrations for the curve pairs from top to bottom are 10 nM, 5 nM, 2.5 nM, 1.25 nM and 0.625 nM, respectively.

FIG. 28: Affinity constant assay of nivolumab to C1q. The antibody concentrations for the curve pairs from top to bottom are 10 nM, 5 nM, 2.5 nM, 1.25 nM and 0.625 nM, respectively.

FIG. 29: Affinity constant assay of VP101(hG1WT) to C1q. The antibody concentrations for the curve pairs from top to bottom are 10 nM, 5 nM, 2.5 nM, 1.25 nM and 0.625 nM, respectively.

FIG. 30: Affinity constant assay of VP101(hG4WT) to C1q. The antigen concentrations for the curve pairs from top to bottom are 10 nM, 5 nM, 2.5 nM, 1.25 nM and 0.625 nM, respectively.

FIG. 31: ADCC activity assay of VP101(hG1WT) and VP101(hG1DM) on a CHO-K1-PD1 target cell system expressing PD-1 antigen.

FIG. 32: CDC activity assay of VP101(hG1WT) and VP101(hG1DM) on a CHO-K1-PD1 target cell system expressing PD-1 antigen.

FIG. 33: Effects of the antibody VP101(hG1DM) on the secretion of cytokine IFN-y induced by PBMC and Raji-PDL1 cell mixed culture as detected by ELISA method.

FIG. 34: Effects of the antibody VP101(hG1DM) on the secretion of cytokine IL-2 induced by PBMC and Raji-PDL1 cell mixed culture as detected by ELISA method.

FIG. 35: ADCP activity assay of VP101(hG1DM) on a CHO-K1-PD1 target cell system expressing PD-1 antigen.

DETAILED DESCRIPTION

The embodiments of the present invention will be described in detail below with reference to the examples. Those skilled in the art will understand that the following examples are only for illustrating the present invention, and should not be construed as limitations on the scope of the present invention. The cases without the specific descriptions of techniques or conditions were carried out according to the technologies or conditions described in the literature in the art (e.g., see, Guide to Molecular Cloning Experiments, authored by J. Sambrook et al., and translated by Huang Peitang et al., third edition, Science Press) or according to the product manual. Reagents or instruments used are all commercially available conventional products if the manufacturers thereof are not specified.

In the following examples of the present invention, the marketed antibody bevacizumab (trade name Avastin®) for the same target was purchased from Roche as a control antibody, or was prepared according to Preparation Example 1.

In the following examples of the present invention, the marketed antibody nivolumab for the same target (trade name Opdivo®) was purchased from BMS as a control antibody.

In the following examples of the present invention, the isotype control antibody used is human anti-hen egg lysozyme IgG (anti-HEL, or human IgG, abbreviated as hIgG) whose variable region sequences are derived from the study entitled "Affinity maturation increases the stability and plasticity of the Fv domain of anti-protein antibodies", published by Acierno et al (Acierno et al., J Mol Biol., 2007; 374(1): 130-46). The hIgG1DM and hIgG4WT used in the examples are isotype control antibodies of anti-HEL with hG1DM and hG4WT constant region sequences. The isotype control antibodies were prepared in the laboratory of Akeso Biopharma, Inc.

Preparation Example 1: Preparation of Anti-VEGFA Antibody Bevacizumab

Chinese Patent Publication CN1259962A is referred to for the amino acid sequences of the heavy chain variable region and the light chain variable region of the marketed anti-VEGFA monoclonal antibody Avastin (bevacizumab). Genscript was entrusted to synthesize nucleotide sequences encoding the heavy chain variable region and the light chain variable region.

Amino acid sequence of the heavy chain variable region of bevacizumab (bevacizumab-Hv): (123 aa)

EVQLVESGGGLVQPGGSLRLSCAASGYTFTNYGMNWVRQAPGKGLEWVGWINTYTG

EPTYAADFKRRFTFSLDTSKSTAYLQMNSLRAEDTAVYYCAKYPHYYGSSHWYFDVW

GQGTLVTVSS (SEQ ID NO: 1)

Nucleotide sequence encoding the heavy chain variable region of bevacizumab: (369 bp)

GAGGTGCAGCTGGTCGAGTCCGGGGGGGGGCTGGTGCAGCCAGGCGGGTCTCTGA

GGCTGAGTTGCGCCGCTTCAGGGTACACCTTCACAAACTATGGAATGAATTGGGTG

CGCCAGGCACCAGGAAAGGGACTGGAGTGGGTCGGCTGGATCAACACTTACACCG

GGGAACCTACCTATGCAGCCGACTTTAAGCGGCGGTTCACCTTCAGCCTGGATACA

AGCAAATCCACTGCCTACCTGCAGATGAACAGCCTGCGAGCTGAGGACACCGCAGT

CTACTATTGTGCTAAATATCCCCACTACTATGGGAGCAGCCATTGGTATTTTGACGT

GTGGGGGCAGGGGACTCTGGTGACAGTGAGCAGC (SEQ ID NO: 2)

Amino acid sequence of the light chain variable region of bevacizumab (bevacizumab-Lv): (107 aa)

DIQMTQSPSSLSASVGDRVTITCSASQDISNYLNWYQQKPGKAPKVLIYFTSSLHSGVPS

RFSGSGSGTDFTLTISSLQPEDFATYYCQQYSTVPWTFGQGTKVEIK (SEQ ID NO: 3)

Nucleotide sequence encoding the light chain variable region of bevacizumab: (321 bp)

GATATTCAGATGACTCAGAGCCCCTCCTCCCTGTCCGCCTCTGTGGGCGACAGGGTC

ACCATCACATGCAGTGCTTCACAGGATATTTCCAACTACCTGAATTGGTATCAGCAG

AAGCCAGGAAAAGCACCCAAGGTGCTGATCTACTTCACTAGCTCCCTGCACTCAGG

AGTGCCAAGCCGGTTCAGCGGATCCGGATCTGGAACCGACTTTACTCTGACCATTTC

TAGTCTGCAGCCTGAGGATTTCGCTACATACTATTGCCAGCAGTATTCTACCGTGCC

ATGGACATTTGGCCAGGGGACTAAAGTCGAGATCAAG (SEQ ID NO: 4)

The heavy chain constant regions were all Ig gamma-1 chain C region, ACCESSION: P01857; the light chain constant regions were all Ig kappa chain C region, ACCESSION: P01834.

The heavy chain cDNA and the light chain cDNA of bevacizumab were cloned into vector pcDNA3.1, and the recombinant expression plasmid of the antibody bevacizumab was obtained. The recombinant plasmid was transfected into 293F cells. The 293F cell culture medium was purified and then detected.

The anti-VEGFA monoclonal antibody Avastin (bevacizumab) was thus obtained.

Preparation Example 2: Sequence Design of Anti-PD-1 Antibody 14C12 and Humanized Antibody 14C12H1L1 thereof and Mutant 14C12H1L1(M)

The amino acid sequences and encoding nucleotide sequences of the heavy and light chains of anti-PD-1 antibody 14C12 and a humanized antibody 14C12H1L1 thereof are identical to those of 14C12 and 14C12H1L1 in Chinese Patent Publication No. CN106967172A, respectively.

(1) Heavy and light chain variable region sequences of 14C12

Amino acid sequence of the heavy chain variable region of 14C12: (118 aa)

EVKLVESGGGLVKPGGSLKLSCAASGFAFSSYDMSWVRQTPEKRLEWVATISGGGRYT
YYPDSVKGRFTISRDNARNTLYLQMSSLRSEDTALYYCANRYGEAWFAYWGQGTLVT
VSA (SEQ ID NO: 5)

Nucleotide sequence encoding the heavy chain variable region of 14C12: (354 bp)

GAGGTCAAACTGGTGGAGAGCGGCGGCGGGCTGGTGAAGCCCGGCGGGTCACTGA
AACTGAGCTGCGCCGCTTCCGGCTTCGCCTTTAGCTCCTACGACATGTCATGGGTGA
GGCAGACCCCTGAGAAGCGCCTGGAATGGGTCGCTACTATCAGCGGAGGCGGGCG
ATACACCTACTATCCTGACTCTGTCAAAGGGAGATTCACAATTAGTCGGGATAACG
CCAGAAATACTCTGTATCTGCAGATGTCTAGTCTGCGGTCCGAGGATACAGCTCTGT
ACTATTGTGCAAACCGGTACGGCGAAGCATGGTTTGCCTATTGGGGACAGGGCACC

CTGGTGACAGTCTCTGCC (SEQ ID NO: 6)

Amino acid sequence of the light chain variable region of 14C12: (107 aa)

DIKMTQSPSSMYASLGERVTFTCKASQDINTYLSWFQQKPGKSPKTLIYRANRLVDGVP
SRFSGSGSGQDYSLTISSLEYEDMGIYYCLQYDEFPLTFGAGTKLELK (SEQ ID NO: 7)

Nucleotide sequence encoding the light chain variable region of 14C12: (321 bp)

GACATTAAGATGACACAGTCCCCTTCCTCAATGTACGCTAGCCTGGGCGAGCGAGT
GACCTTCACATGCAAAGCATCCCAGGACATCAACACATACCTGTCTTGGTTTCAGCA
GAAGCCAGGCAAAAGCCCCAAGACCCTGATCTACCGGGCCAATAGACTGGTGGAC
GGGGTCCCCAGCAGATTCTCCGGATCTGGCAGTGGGCAGGATTACTCCCTGACCAT
CAGCTCCCTGGAGTATGAAGACATGGGCATCTACTATTGCCTGCAGTATGATGAGTT
CCCTCTGACCTTTGGAGCAGGCACAAAACTGGAACTGAAG (SEQ ID NO: 8)

(2) Heavy and light chain variable region and heavy and light chain sequences of humanized monoclonal antibody 14C12H1L1

Amino acid sequence of the heavy chain variable region of 14C12H1L1: (118 aa)

EVQLVESGGGLVQPGGSLRLSCAASGFAFSSYDMSWVRQAPGKGLDWVATISGGGRY TYYPDSVKGRFTISRDNSKNNLYLQMNSLRAEDTALYYCANRYGEAWFAYWGQGTLV TVSS (SEQ ID NO: 9)

Nucleotide sequence encoding the heavy chain variable region of 14C12H1L1: (354 bp)

GAAGTGCAGCTGGTCGAGTCTGGGGGAGGGCTGGTGCAGCCCGGCGGGTCACTGCG ACTGAGCTGCGCAGCTTCCGGATTCGCCTTTAGCTCCTACGACATGTCCTGGGTGCG ACAGGCACCAGGAAAGGGACTGGATTGGGTCGCTACTATCTCAGGAGGCGGGAGA TACACCTACTATCCTGACAGCGTCAAGGGCCGGTTCACAATCTCTAGAGATAACAG TAAGAACAATCTGTATCTGCAGATGAACAGCCTGAGGGCTGAGGACACCGCACTGT ACTATTGTGCCAACCGCTACGGGGAAGCATGGTTTGCCTATTGGGGGCAGGGAACC CTGGTGACAGTCTCTAGT (SEQ ID NO: 10)

Amino acid sequence of the light chain variable region of 14C12H1L1: (107 aa)

DIQMTQSPSSMSASVGDRVTFTCRASQDINTYLSWFQQKPGKSPKTLIYRANRLVSGVP SRFSGSGSGQDYTLTISSLQPEDMATYYCLQYDEFPLTFGAGTKLELK (SEQ ID NO: 11)

Nucleotide sequence encoding the light chain variable region of 14C12H1L1: (321 bp)

GACATTCAGATGACTCAGAGCCCCTCCTCCATGTCCGCCTCTGTGGGCGACAGGGTC ACCTTCACATGCCGCGCTAGTCAGGATATCAACACCTACCTGAGCTGGTTTCAGCAG AAGCCAGGGAAAAGCCCCAAGACACTGATCTACCGGGCTAATAGACTGGTGTCTGG AGTCCCAAGTCGGTTCAGTGGCTCAGGGAGCGGACAGGACTACACTCTGACCATCA GCTCCCTGCAGCCTGAGGACATGGCAACCTACTATTGCCTGCAGTATGATGAGTTCC CACTGACCTTTGGCGCCGGGACAAAACTGGAGCTGAAG (SEQ ID NO: 12)

Amino acid sequence of the heavy chain (14C12H1) of 14C12H1L1: (448 aa)

EVQLVESGGGLVQPGGSLRLSCAASGFAFSSYDMSWVRQAPGKGLDWVATISGGGRY

TYYPDSVKGRFTISRDNSKNNLYLQMNSLRAEDTALYYCANRYGEAWFAYWGQGTLV

TVSSASTKGPSVFPLAPSSKSTSGGTAALGCLVKDYFPEPVTVSWNSGALTSGVHTFPA

VLQSSGLYSLSSVVTVPSSSLGTQTYICNVNHKPSNTKVDKKVEPKSCDKTHTCPPCPAP

ELLGGPSVFLFPPKPKDTLMISRTPEVTCVVVDVSHEDPEVKFNWYVDGVEVHNAKTK

PREEQYNSTYRVVSVLTVLHQDWLNGKEYKCKVSNKALPAPIEKTISKAKGQPREPQV

YTLPPSRDELTKNQVSLTCLVKGFYPSDIAVEWESNGQPENNYKTTPPVLDSDGSFFLY

SKLTVDKSRWQQGNVFSCSVMHEALHNHYTQKSLSLSPGK (SEQ ID NO: 13)

Nucleotide sequence encoding the heavy chain (14C12H1) of 14C12H1L1: (1344 bp)

GAAGTGCAGCTGGTCGAGTCTGGGGGAGGGCTGGTGCAGCCCGGCGGGTCACTGCG

ACTGAGCTGCGCAGCTTCCGGATTCGCCTTTAGCTCCTACGACATGTCCTGGGTGCG

ACAGGCACCAGGAAAGGGACTGGATTGGGTCGCTACTATCTCAGGAGGCGGGAGA

TACACCTACTATCCTGACAGCGTCAAGGGCCGGTTCACAATCTCTAGAGATAACAG

TAAGAACAATCTGTATCTGCAGATGAACAGCCTGAGGGCTGAGGACACCGCACTGT

ACTATTGTGCCAACCGCTACGGGGAAGCATGGTTTGCCTATTGGGGGCAGGGAACC

CTGGTGACAGTCTCTAGTGCCAGCACCAAAGGACCTAGCGTGTTTCCTCTCGCCCCC

TCCTCCAAAAGCACCAGCGGAGGAACCGCTGCTCTCGGATGTCTGGTGAAGGACTA

CTTCCCTGAACCCGTCACCGTGAGCTGGAATAGCGGCGCTCTGACAAGCGGAGTCC

ATACATTCCCTGCTGTGCTGCAAAGCAGCGGACTCTATTCCCTGTCCAGCGTCGTCA
CAGTGCCCAGCAGCAGCCTGGGCACCCAGACCTACATCTGTAACGTCAACCACAAG
CCCTCCAACACCAAGGTGGACAAGAAAGTGGAGCCCAAATCCTGCGACAAGACAC
ACACCTGTCCCCCCTGTCCTGCTCCGAACTCCTCGGAGGCCCTAGCGTCTTCCTCTT
TCCTCCCAAACCCAAGGACACCCTCATGATCAGCAGAACCCCTGAAGTCACCTGTG
TCGTCGTGGATGTCAGCCATGAGGACCCCGAGGTGAAATTCAACTGGTATGTCGAT
GGCGTCGAGGTGCACAACGCCAAAACCAAGCCCAGGGAGGAACAGTACAACTCCA
CCTACAGGGTGGTGTCCGTGCTGACAGTCCTCCACCAGGACTGGCTGAACGGCAAG
GAGTACAAGTGCAAGGTGTCCAACAAGGCTCTCCCTGCCCCCATTGAGAAGACCAT
CAGCAAGGCCAAAGGCCAACCCAGGGAGCCCCAGGTCTATACACTGCCTCCCTCCA
GGGACGAACTCACCAAGAACCAGGTGTCCCTGACCTGCCTGGTCAAGGGCTTTTAT
CCCAGCGACATCGCCGTCGAGTGGGAGTCCAACGGACAGCCCGAGAATAACTACA
AGACCACCCCTCCTGTCCTCGACTCCGACGGCTCCTTCTTCCTGTACAGCAAGCTGA
CCGTGGACAAAAGCAGGTGGCAGCAGGGAAACGTGTTCTCCTGCAGCGTGATGCAC
GAAGCCCTCCACAACCACTACACCCAGAAAAGCCTGTCCCTGAGCCCCGGCAAA

(SEQ ID NO: 14)

Amino acid sequence of the light chain (14C12L1) of 14C12H1L1: (214 aa)

DIQMTQSPSSMSASVGDRVTFTCRASQDINTYLSWFQQKPGKSPKTLIYRANRLVSGVP
SRFSGSGSGQDYTLTISSLQPEDMATYYCLQYDEFPLTFGAGTKLELKRTVAAPSVFIFP
PSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 15)

Nucleotide sequence encoding the light chain (14C12L1) of 14C12H1L1: (642 bp)

GACATTCAGATGACTCAGAGCCCCTCCTCCATGTCCGCCTCTGTGGGCGACAGGGTC
ACCTTCACATGCCGCGCTAGTCAGGATATCAACACCTACCTGAGCTGGTTTCAGCAG
AAGCCAGGGAAAAGCCCCAAGACACTGATCTACCGGGCTAATAGACTGGTGTCTGG
AGTCCCAAGTCGGTTCAGTGGCTCAGGGAGCGGACAGGACTACACTCTGACCATCA
GCTCCCTGCAGCCTGAGGACATGGCAACCTACTATTGCTGCAGTATGATGAGTTCC
CACTGACCTTTGGCGCCGGGACAAAACTGGAGCTGAAGCGAACTGTGGCCGCTCCC

TCCGTCTTCATTTTTCCCCCTTCTGACGAACAGCTGAAATCAGGCACAGCCAGCGTG

GTCTGTCTGCTGAACAATTTCTACCCTAGAGAGGCAAAAGTGCAGTGGAAGGTCGA

TAACGCCCTGCAGTCCGGCAACAGCCAGGAGAGTGTGACTGAACAGGACTCAAAA

GATAGCACCTATTCCCTGTCTAGTACACTGACTCTGTCCAAGGCTGATTACGAGAAG

CACAAAGTGTATGCATGCGAAGTGACACATCAGGGACTGTCAAGCCCCGTGACTAA

GTCTTTTAACCGGGGCGAATGT (SEQ ID NO: 16)

(3) Heavy and light chain variable region sequences of 14C12H1L1(M)

Individual amino acids in the framework region (light chain) were mutated on the basis of 14C12H1L1 to obtain 14C12H1L1(M).

Heavy chain variable region 14C12H1(M) of 14C12H1L1(M):
is identical with the heavy chain variable region 14C12H1 of 14C12H1L1, namely, the amino acid sequence is set forth in SEQ ID NO: 9.

Light chain variable region 14C12L1(M) of 14C12H1L1(M): (108 aa, mutation positions underlined in the amino acid sequence based on 14C12H1L1)

DIQMTQSPSSMSASVGDRVTFTCRASQDINTYLSWFQQKPGKSPKTLIYRANRLVSGVP

SRFSGSGSGQDYTLTISSLQPEDMATYYCLQYDEFPLTFGAGTKLELK<u>R</u>  (SEQ ID NO:

17)

Preparation Example 3: Sequence Design of Bispecific Antibodies

1. Sequence design

The structure of the bispecific antibody of the present invention is in the Morrison form (IgG-scFv), i.e., C-termini of two heavy chains of an IgG antibody are each linked to a scFv fragment of another antibody, and the main composition design of the heavy and light chains is as shown in Table 1 below.

On the basis of bevacizumab, the VP101 antibody, in which amino acid sequences of the heavy chain variable region and the light chain variable region of 14C12H1L1(M) are taken as a ScFv fragment part, is referred to as VP101(M). Compared to 14C12H1L1, 14C12H1L1(M) effectively optimized the structure of the bispecific antibody and improved the effectiveness.

Table 1: Compositional Design of Heavy and Light Chains of VP101(M) and VP101(G4M)

| Bispecific antibody No. | Immunoglobulin moiety | | Linker fragment | scFv part |
| --- | --- | --- | --- | --- |
| | Heavy chain | Light chain | | |
| VP101(M) | Bevacizum ab-H | Bevacizumab-L | Linker1 | 14C12H1(M)$_V$- Linkerl-14C12L1(M)$_V$ |

(continued)

| Bispecific antibody No. | Immunoglobulin moiety | | Linker fragment | scFv part |
|---|---|---|---|---|
| | Heavy chain | Light chain | | |
| VP101(G4M) | Bevacizum ab-G4H | Bevacizumab-L | Linker1 | 14C12H1(M)$_V$- Linkerl-14C12L1(M)$_V$ |

In Table 1 above:
(1) Those with "V" labeled at lower right corner refer to the variable region of corresponding heavy chain or the variable region of corresponding light chain. For those without "V" label, the corresponding heavy or light chain is the full length comprising the constant region. The corresponding sequences described in the above preparation examples are referred to for the amino acid sequences of these variable regions or the full length and the nucleotide sequences encoding them.
(2) The amino acid sequence of Linker 1 is GGGGSGGGGSGGGGSG
GGGS (SEQ ID NO: 18)
Optionally, the amino acid sequence GGGGSGGGGSGGGGS (SEQ ID NO: 19) can be used as Linker 2 in place of the aforementioned Linker 1.
(3) Bevacizumab-H used Ig gamma-1 chain C region (ACCESSION: P01857) as the heavy chain constant region.
(4) Bevacizumab-G4H used Ig gamma-4 chain C region (ACCESSION: P01861.1) as the heavy chain constant region.

2. Expression and purification of antibody VP101(M)

The heavy chain cDNA sequence and the light chain cDNA sequence of VP101 were each cloned into vector pUC57simple (provided by Genscript) to obtain plasmids pUC57simple-VP101H and pUC57simple-VP101L, respectively.

Plasmids pUC57simple-VP101H and pUC57simple-VP101L were enzyme-digested (HindIII&EcoRI), and heavy and light chains isolated by electrophoresis were subcloned into vector pcDNA3.1, and recombinant plasmids were extracted to co-transfect 293F cells. After 7 days of cell culture, the culture medium was centrifuged at high speed, and the supernatant was concentrated and loaded onto a HiTrap MabSelect SuRe column. The protein was further eluted in one step with Elution Buffer, and the target sample antibody VP101 was isolated and buffer exchanged into PBS.

3. Detection of antibody VP101(M)

The purified sample was added to both a reduced protein electrophoresis loading buffer and a non-reduced protein electrophoresis loading buffer, and then boiled for SDS-PAGE electrophoresis detection.

For differentiation from the mutated antibody of Preparation Example 4, VP101(M) is also referred to as VP101(hG1WT) in the present invention. VP101(M) described above is the "wild-type", comprising an Ig gamma-1 chain C region (ACCESSION: P01857) as the heavy chain constant region and an Ig kappa chain C region (ACCESSION: P01834) as the light chain constant region.

Amino acid sequence of the heavy chain of the immunoglobulin moiety in VP101(hG1WT): (453 aa)

EVQLVESGGGLVQPGGSLRLSCAASGYTFTNYGMNWVRQAPGKGLEWVGWINTYTG

EPTYAADFKRRFTFSLDTSKSTAYLQMNSLRAEDTAVYYCAKYPHYYGSSHWYFDVW

GQGTLVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCDKTHT

CPPCPAPELLGGPSVFLFPPKPKDTLMISRTPEVTCVVVDVSHEDPEVKFNWYVDGVEV

HNAKTKPREEQYNSTYRVVSVLTVLHQDWLNGKEYKCKVSNKALPAPIEKTISKAKGQ

PREPQVYTLPPSRDELTKNQVSLTCLVKGFYPSDIAVEWESNGQPENNYKTTPPVLDSD

GSFFLYSKLTVDKSRWQQGNVFSCSVMHEALHNHYTQKSLSLSPGK (SEQ ID NO: 20)

Nucleotide sequence encoding the heavy chain of the immunoglobulin moiety in VP101(hG1WT): (1359 bp)

GAGGTGCAGCTGGTCGAGTCCGGGGGGGGGCTGGTGCAGCCAGGCGGGTCTCTGA

GGCTGAGTTGCGCCGCTTCAGGGTACACCTTCACAAACTATGGAATGAATTGGGTG

CGCCAGGCACCAGGAAAGGGACTGGAGTGGGTCGGCTGGATCAACACTTACACCG

GGGAACCTACCTATGCAGCCGACTTTAAGCGGCGGTTCACCTTCAGCCTGGATACA

AGCAAATCCACTGCCTACCTGCAGATGAACAGCCTGCGAGCTGAGGACACCGCAGT

CTACTATTGTGCTAAATATCCCCACTACTATGGGAGCAGCCATTGGTATTTTGACGT

GTGGGGGCAGGGGACTCTGGTGACAGTGAGCAGCGCAAGCACCAAAGGGCCCAGC

GTGTTTCCTCTCGCCCCCTCCTCCAAAAGCACCAGCGGAGGAACCGCTGCTCTCGGA

TGTCTGGTGAAGGACTACTTCCCTGAACCCGTCACCGTGAGCTGGAATAGCGGCGC

TCTGACAAGCGGAGTCCATACATTCCCTGCTGTGCTGCAAAGCAGCGGACTCTATTC

CCTGTCCAGCGTCGTCACAGTGCCCAGCAGCAGCCTGGGCACCCAGACCTACATCT

GTAACGTCAACCACAAGCCCTCCAACACCAAGGTGGACAAGAAAGTGGAGCCCAA

ATCCTGCGACAAGACACACACCTGTCCCCCTGTCCTGCTCCCGAACTCCTCGGAGG

CCCTAGCGTCTTCCTCTTTCCTCCCAAACCCAAGGACACCCTCATGATCAGCAGAAC

CCCTGAAGTCACCTGTGTCGTCGTGGATGTCAGCCATGAGGACCCCGAGGTGAAAT

TCAACTGGTATGTCGATGGCGTCGAGGTGCACAACGCCAAAACCAAGCCCAGGGAG

GAACAGTACAACTCCACCTACAGGGTGGTGTCCGTGCTGACAGTCCTCCACCAGGA

CTGGCTGAACGGCAAGGAGTACAAGTGCAAGGTGTCCAACAAGGCTCTCCCTGCCC

CCATTGAGAAGACCATCAGCAAGGCCAAAGGCCAACCCAGGGAGCCCCAGGTCTA

TACACTGCCTCCCTCCAGGGACGAACTCACCAAGAACCAGGTGTCCCTGACCTGCC

TGGTCAAGGGCTTTTATCCCAGCGACATCGCCGTCGAGTGGGAGTCCAACGGACAG

CCCGAGAATAACTACAAGACCACCCCTCCTGTCCTCGACTCCGACGGCTCCTTCTTC

CTGTACAGCAAACTGACCGTCGATAAATCTAGGTGGCAGCAGGGCAACGTGTTCTC

TTGTTCCGTGATGCATGAAGCACTGCACAACCATTATACCCAGAAGTCTCTGAGCCT

GTCCCCCGGCAAG (SEQ ID NO: 21)

For differentiation from the mutated antibody of Preparation Example 4, VP101(G4M) is also referred to as VP101(hG4WT) in the present invention. VP101(G4M) described above is the "wild-type", comprising an Ig gamma-4 chain C region (ACCESSION: P01861.1) as the heavy chain constant region and an Ig kappa chain C region (ACCESSION: P01834) as the light chain constant region.

Amino acid sequence of the heavy chain of the immunoglobulin moiety in VP101(hG4WT): (450 aa)

EVQLVESGGGLVQPGGSLRLSCAASGYTFTNYGMNWVRQAPGKGLEWVGWINTYTG
EPTYAADFKRRFTFSLDTSKSTAYLQMNSLRAEDTAVYYCAKYPHYYGSSHWYFDVW
GQGTLVTVSSASTKGPSVFPLAPCSRSTSESTAALGCLVKDYFPEPVTVSWNSGALTSG
VHTFPAVLQSSGLYSLSSVVTVPSSSLGTKTYTCNVDHKPSNTKVDKRVESKYGPPCPP
CPAPEFLGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVLDSDGS
FFLYSRLTVDKSRWQEGNVFSCSVMHEALHNHYTQKSLSLSLGK (SEQ ID NO: 22)

Nucleotide sequence encoding the heavy chain of the immunoglobulin moiety in VP101(hG4WT): (1350 bp)

GAGGTGCAGCTGGTCGAGTCCGGGGGGGGGCTGGTGCAGCCAGGCGGGTCTCTGA
GGCTGAGTTGCGCCGCTTCAGGGTACACCTTCACAAACTATGGAATGAATTGGGTG
CGCCAGGCACCAGGAAAGGGACTGGAGTGGGTCGGCTGGATCAACACTTACACCG
GGGAACCTACCTATGCAGCCGACTTTAAGCGGCGGTTCACCTTCAGCCTGGATACA
AGCAAATCCACTGCCTACCTGCAGATGAACAGCCTGCGAGCTGAGGACACCGCAGT
CTACTATTGTGCTAAATATCCCCACTACTATGGGAGCAGCCATTGGTATTTTGACGT
GTGGGGGCAGGGGACTCTGGTGACAGTGAGCAGCGCAAGCACCAAAGGGCCCTCG
GTCTTCCCCCTGGCGCCCTGCTCCAGGAGCACCTCCGAGAGCACAGCCGCCCTGGG
CTGCCTGGTCAAGGACTACTTCCCCGAACCGGTGACGGTGTCGTGGAACTCAGGCG
CCCTGACCAGCGGCGTGCACACCTTCCCGGCTGTCCTACAGTCCTCAGGACTCTACT
CCCTCAGCAGCGTGGTGACCGTGCCCTCCAGCAGCTTGGGCACGAAGACCTACACC
TGCAACGTAGATCACAAGCCCAGCAACACCAAGGTGGACAAGAGAGTTGAGTCCA
AATATGGTCCCCCATGCCCACCATGCCCAGCACCTGAGTTCCTGGGGGGACCATCA
GTCTTCCTGTTCCCCCAAAACCCAAGGACACTCTCATGATCTCCCGGACCCCTGAG

GTCACGTGCGTGGTGGTGGACGTGAGCCAGGAAGACCCCGAGGTCCAGTTCAACTG
GTACGTGGATGGCGTGGAGGTGCATAATGCCAAGACAAAGCCGCGGGAGGAGCAG
TTCAACAGCACGTACCGTGTGGTCAGCGTCCTCACCGTCCTGCACCAGGACTGGCTG
AACGGCAAGGAGTACAAGTGCAAGGTCTCCAACAAAGGCCTCCCGTCCTCCATCGA
GAAAACCATCTCCAAAGCCAAAGGGCAGCCCCGAGAGCCACAGGTGTACACCCTG
CCCCCATCCCAGGAGGAGATGACCAAGAACCAGGTCAGCCTGACCTGCCTGGTCAA
AGGCTTCTACCCCAGCGACATCGCCGTGGAGTGGGAGAGCAATGGGCAGCCGGAG
AACAACTACAAGACCACGCCTCCCGTGCTGGACTCCGACGGCTCCTTCTTCCTCTAC
AGCAGGCTAACCGTGGACAAGAGCAGGTGGCAGGAGGGGAATGTCTTCTCATGCTC
CGTGATGCATGAGGCTCTGCACAACCACTACACACAGAAGTCTCTGAGCCTGTCCC
TCGGCAAG (SEQ ID NO: 23)

Preparation Example 4: Non-Variable Region Amino Acid Mutation Design Based on Humanized Bispecific Antibody VP101(hG1WT)

On the basis of VP101(hG1WT) obtained in Preparation Example 3, VP101(hG1DM) was obtained by introducing a leucine-to-alanine point mutation at position 234 (L234A) and a leucine-to-alanine point mutation at position 235 (L235A) in the heavy chain.

Amino acid sequence of the heavy chain of the immunoglobulin moiety in VP101(hG1DM): (453 aa, mutation positions underlined)

EVQLVESGGGLVQPGGSLRLSCAASGYTFTNYGMNWVRQAPGKGLEWVGWINTYTG
EPTYAADFKRRFTFSLDTSKSTAYLQMNSLRAEDTAVYYCAKYPHYYGSSHWYFDVW
GQGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTHT
CPPCPAPEAAGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAKGQ
PREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 24)

Nucleotide sequence encoding the heavy chain of the immunoglobulin moiety in VP101(hG1DM): (1359 bp, mutation positions underlined)

GAGGTGCAGCTGGTCGAGTCCGGGGGGGGGCTGGTGCAGCCAGGCGGGTCTCTGA
GGCTGAGTTGCGCCGCTTCAGGGTACACCTTCACAAACTATGGAATGAATTGGGTG
CGCCAGGCACCAGGAAAGGGACTGGAGTGGGTCGGCTGGATCAACACTTACACCG
GGGAACCTACCTATGCAGCCGACTTTAAGCGGCGGTTCACCTTCAGCCTGGATACA
AGCAAATCCACTGCCTACCTGCAGATGAACAGCCTGCGAGCTGAGGACACCGCAGT
CTACTATTGTGCTAAATATCCCCACTACTATGGGAGCAGCCATTGGTATTTTGACGT
GTGGGGGCAGGGGACTCTGGTGACAGTGAGCAGCGCAAGCACCAAAGGGCCCAGC
GTGTTTCCTCTCGCCCCCTCCTCCAAAAGCACCAGCGGAGGAACCGCTGCTCTCGGA
TGTCTGGTGAAGGACTACTTCCCTGAACCCGTCACCGTGAGCTGGAATAGCGGCGC
TCTGACAAGCGGAGTCCATACATTCCCTGCTGTGCTGCAAAGCAGCGGACTCTATTC
CCTGTCCAGCGTCGTCACAGTGCCCAGCAGCAGCCTGGGCACCCAGACCTACATCT
GTAACGTCAACCACAAGCCCTCCAACACCAAGGTGGACAAGAAAGTGGAGCCCAA
ATCCTGCGACAAGACACACACCTGTCCCCCCTGTCCTGCTCCCGAA<u>GCTGCT</u>GGAG
GCCCTAGCGTCTTCCTCTTTCCTCCCAAACCCAAGGACACCCTCATGATCAGCAGAA
CCCCTGAAGTCACCTGTGTCGTCGTGGATGTCAGCCATGAGGACCCCGAGGTGAAA
TTCAACTGGTATGTCGATGGCGTCGAGGTGCACAACGCCAAAACCAAGCCCAGGGA
GGAACAGTACAACTCCACCTACAGGGTGGTGTCCGTGCTGACAGTCCTCCACCAGG
ACTGGCTGAACGGCAAGGAGTACAAGTGCAAGGTGTCCAACAAGGCTCTCCCTGCC
CCCATTGAGAAGACCATCAGCAAGGCCAAAGGCCAACCCAGGGAGCCCCAGGTCT
ATACACTGCCTCCCTCCAGGGACGAACTCACCAAGAACCAGGTGTCCCTGACCTGC
CTGGTCAAGGGCTTTTATCCCAGCGACATCGCCGTCGAGTGGGAGTCCAACGGACA
GCCCGAGAATAACTACAAGACCACCCCTCCTGTCCTCGACTCCGACGGCTCCTTCTT
CCTGTACAGCAAACTGACCGTCGATAAATCTAGGTGGCAGCAGGGCAACGTGTTCT
CTTGTTCCGTGATGCATGAAGCACTGCACAACCATTATACCCAGAAGTCTCTGAGCC
TGTCCCCCGGCAAG (SEQ ID NO: 25)

The amino acid sequences and encoding nucleotide sequences of the light chain of the immunoglobulin moiety of VP101(hG1DM), VP101(hG1WT) and VP101(hG4WT) are identical.

Amino acid sequence of the light chain of the immunoglobulin moiety in VP101(hG1DM): (214 aa)

DIQMTQSPSSLSASVGDRVTITCSASQDISNYLNWYQQKPGKAPKVLIYFTSSLHSGVPS

RFSGSGSGTDFTLTISSLQPEDFATYYCQQYSTVPWTFGQGTKVEIKRTVAAPSVFIFPPS

DEQLKSGTASVVCLLNNFYPREAKVQWKVDNALQSGNSQESVTEQDSKDSTYSLSSTL

TLSKADYEKHKVYACEVTHQGLSSPVTKSFNRGEC (SEQ ID NO: 26)

Nucleotide sequence encoding the light chain of the immunoglobulin moiety in VP101(hG1DM): (642 bp)

GATATTCAGATGACTCAGAGCCCCTCCTCCCTGTCCGCCTCTGTGGGCGACAGGGTC

ACCATCACATGCAGTGCTTCACAGGATATTTCCAACTACCTGAATTGGTATCAGCAG

AAGCCAGGAAAAGCACCCAAGGTGCTGATCTACTTCACTAGCTCCCTGCACTCAGG

AGTGCCAAGCCGGTTCAGCGGATCCGGATCTGGAACCGACTTTACTCTGACCATTTC

TAGTCTGCAGCCTGAGGATTTCGCTACATACTATTGCCAGCAGTATTCTACCGTGCC

ATGGACATTTGGCCAGGGGACTAAAGTCGAGATCAAGCGGACCGTGGCCGCTCCCA

GTGTCTTCATTTTTCCCCCTAGCGACGAACAGCTGAAATCCGGGACAGCCTCTGTGG

TCTGTCTGCTGAACAACTTCTACCCTAGAGAGGCAAAAGTGCAGTGGAAGGTCGAT

AACGCCCTGCAGAGTGGCAATTCACAGGAGAGCGTGACAGAACAGGACTCCAAAG

ATTCTACTTATAGTCTGTCAAGCACACTGACTCTGAGCAAGGCTGACTACGAAAAG

CATAAAGTGTATGCATGTGAGGTCACCCACCAGGGGCTGAGCAGTCCAGTCACCAA

GTCATTCAACAGAGGCGAGTGC (SEQ ID NO: 27)

Example 1: Affinity Constant Assay of FcγRI to VP101(hG1WT) and VP101(hG1DM)

The Fc receptor FcγRI, also known as CD64, can bind to the Fc fragment of IgG antibodies and is involved in antibody-dependent cell-mediated cytotoxicity (ADCC). The binding capacity of a therapeutic monoclonal antibody to Fc receptors will influence the safety and efficacy of the antibody.

The affinity constants of VP101(hG1WT) and VP101(hG1DM) to FcγRI were determined using a Fortebio Octet system in this experiment to evaluate the ADCC activity of each antibody.

The method for determining the affinity constants of the antibodies to FcγRI using the Fortebio Octet system is briefly described as follows: a sample dilution buffer was a solution of PBS, 0.02% Tween-20 and 0.1% BSA, pH 7.4. 1 μg/mL FcγRIa was immobilized on an HIS1K sensor for 50 s. The sensor was equilibrated in a buffer for 60 s, and the binding of the immobilized CD64 on the sensor to the antibodies at concentrations of 3.12-50 nM (serial two-fold dilution) was determined for 120 s. The antibodies were dissociated in the buffer for 120 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

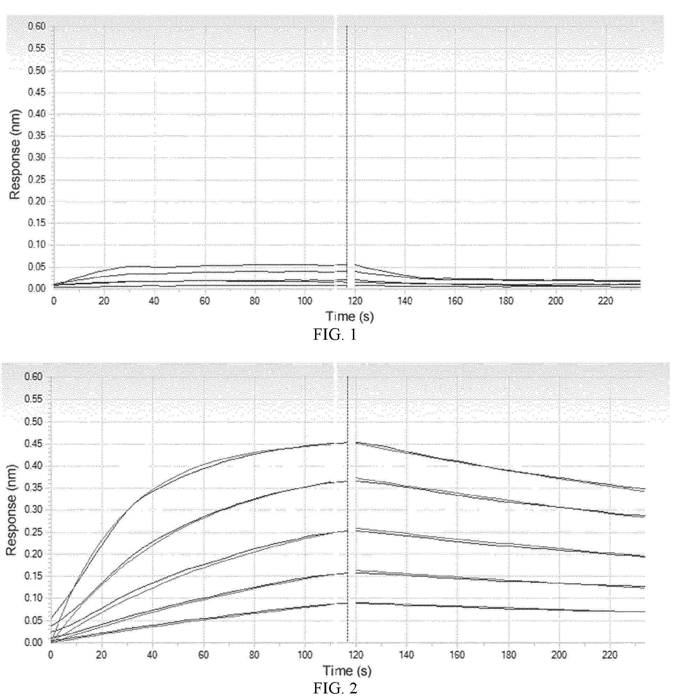

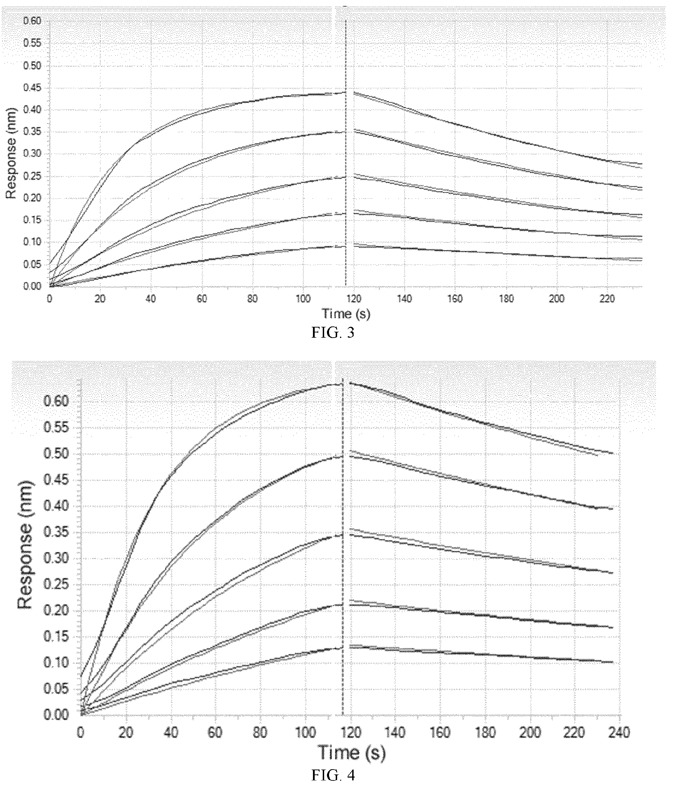

The affinity constants of FcγRI to VP101(hG1WT), VP101(hG4WT) and VP101(hG1DM) as well as control antibodies nivolumab and bevacizumab are shown in Table 1 and FIGs. 1-5.

Table 1: Kinetics for Binding of VP101(hG1WT) and VP101(hG1DM) and Isotypes Thereof to FcγRI

| Antibody | $K_D$ (M) | kon (1/Ms) | SE (kon) | kdis (1/s) | SE (kdis) | Rmax (nm) |
|---|---|---|---|---|---|---|
| VP101(hG1DM) | N/A | N/A | N/A | N/A | N/A | N/A |
| Bevacizumab | 3.68E-09 | 6.61E+05 | 2.07E+04 | 2.44E-03 | 9.02E-05 | 0.46-0.49 |
| Nivolumab | 6.20E-09 | 6.98E+05 | 2.22E+04 | 4.32E-03 | 9.88E-05 | 0.48-0.53 |

(continued)

| Antibody | $K_D$ (M) | kon (1/Ms) | SE (kon) | kdis (1/s) | SE (kdis) | Rmax (nm) |
|---|---|---|---|---|---|---|
| VP101(hG1WT) | 3.95E-09 | 5.67E+05 | 1.82E+04 | 2.24E-03 | 9.02E-05 | 0.68-0.80 |
| VP101(hG4WT) | 8.52E-09 | 6.28E+05 | 2.12E+04 | 5.35E-03 | 1.07E-04 | 0.61-0.65 |

N/A indicates that the antibody had no binding or an extremely weak binding signal to the antigen, and thus the results were not analyzed and no corresponding data was obtained.

The results show that VP101(hG1WT) can bind to FcγRI with an affinity constant of 3.95E-09M; VP101(hG4WT) can bind to FcγRI with an affinity constant of 8.52E-09M; bevacizumab can bind to FcγRI with an affinity constant of 3.68E-09M; nivolumab can bind to FcγRI with an affinity constant of 6.20E-09M; VP101(hG1DM) had no binding or an extremely weak binding signal to FcγRI, and thus the results were not analyzed and no corresponding data were obtained. The results show that the affinity of other antibodies to FcγRI is similar except for no binding of VP101(hG1DM) to FcγRI. The binding activity of VP101(hG1DM) was effectively eliminated.

Example 2: Affinity Constant Assay of FcγRIIa H131 to VP101(hG1WT) and VP101(hG1DM)

The Fc receptor FcγRIIa_H131 (also known as CD32a_H131), can bind to the Fc fragment of IgG antibodies and mediate ADCC effects.

The affinity constants of VP101(hG1WT) and VP101(hG1DM) to FcγRIIa_H131 were determined using a Fortebio Octet system in this experiment to evaluate the ADCC activity of each antibody.

The method for determining the affinity constants of VP101(hG1WT) and VP101(hG1DM) to FcγRIIa_H131 using a Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcγRIIa_H131 was immobilized on an NTA sensor for an immobilization time of 60 s. The sensor was equilibrated in a buffer of PBS, 0.02% Tween-20, 0.02% casein and 0.1% BSA (pH 7.4) for 600 s of blocking, and the binding of the immobilized FcγRIIa_H131 on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

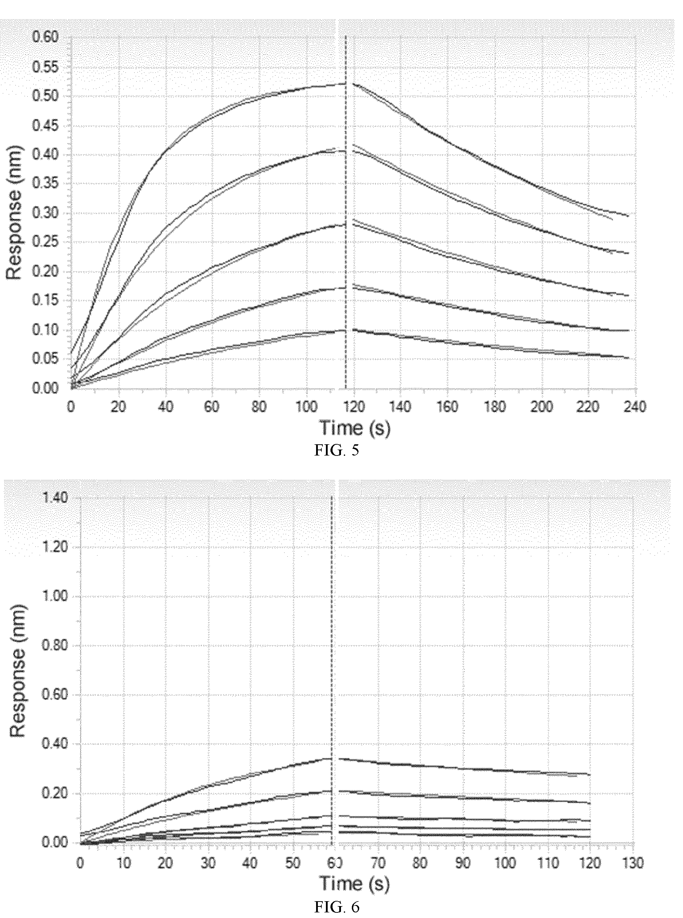

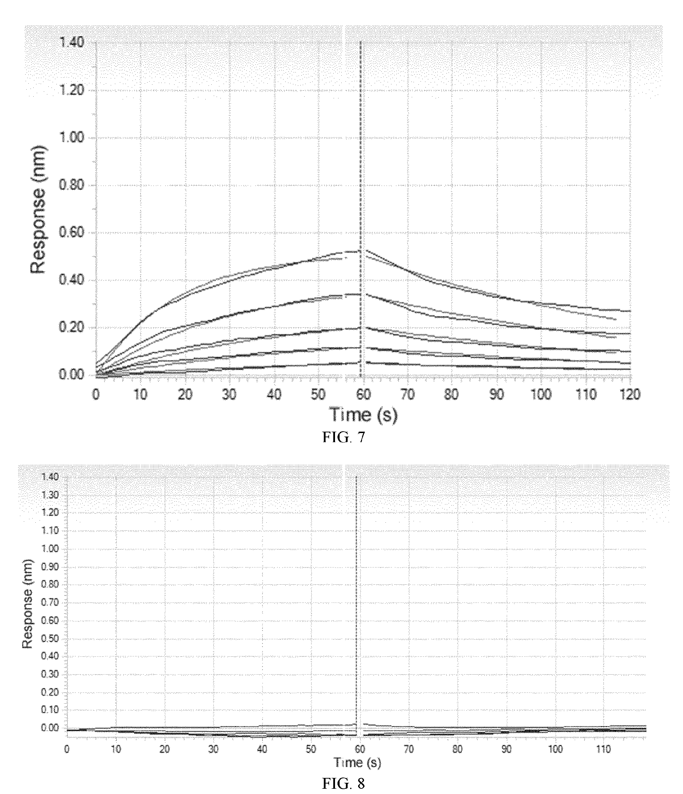

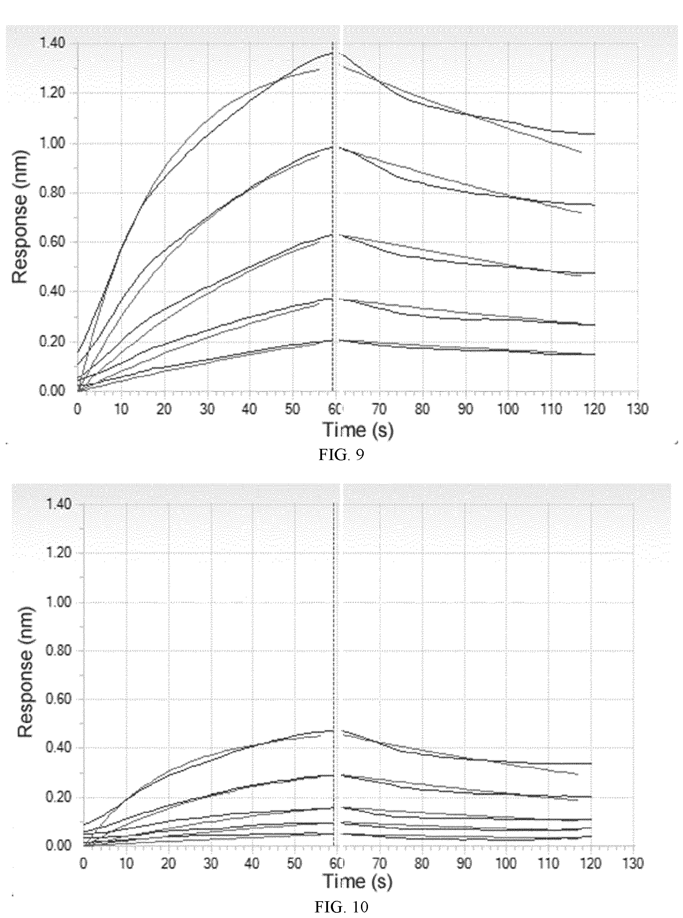

The affinity constants of FcγRIIa_H131 to VP101(hG1WT), VP101(hG4WT) and VP101(hG1DM) as well as control antibodies nivolumab and bevacizumab are shown in Table 2 and FIGs. 6-10.

Table 2: Kinetics for Binding of VP101(hG1WT) and VP101(hG1DM) and Isotypes Thereof to FcγRIIa_H131

| Antibody | $K_D$ (M) | kon (1/Ms) | SE (kon) | kdis (1/s) | SE (kdis) | Rmax (nm) |
|---|---|---|---|---|---|---|
| VP101(hG1DM) | 3.57E-08 | 1.15E+05 | 1.01E+04 | 4.11E-03 | 3.27E-04 | 0.41-0.53 |
| Bevacizumab | 6.44E-08 | 2.10E+05 | 1.78E+04 | 1.36E-02 | 5.14E-04 | 0.49-0.68 |
| Nivolumab | N/A | N/A | N/A | N/A | N/A | N/A |
| VP101(hG1WT) | 2.28E-08 | 2.41E+05 | 1.48E+04 | 5.50E-03 | 3.49E-04 | 1.40-1.52 |
| VP101(hG4WT) | 3.68E-08 | 2.13E+05 | 2.43E+04 | 7.83E-03 | 6.65E-04 | 0.41-0.57 |

N/A indicates that the antibody had no binding or an extremely weak binding signal to the antigen, and thus the results were not analyzed and no corresponding data was obtained.

The results show that VP101(hG1WT) can bind to FcγRIIa_H131 with an affinity constant of 2.28E-08M; VP101(hG4WT) can bind to FcγRIIa_H131 with an affinity constant of 3.68E-08M; bevacizumab can bind to FcγRIIa_H131 with an affinity constant of 6.44E-08M; VP101(hG1DM) can bind to FcγRIIa_H131 with an affinity constant of 3.57E-08M; while nivolumab had no binding or an extremely weak binding signal to FcγRIIa_H131, and thus the results were not analyzed and no corresponding data were obtained.

The results show that, except for no binding of nivolumab to FcγRIIa_H131, other antibodies bound to FcγRIIa_H131 with the following strong-to-weak affinities: VP101(hG1WT), VP101(hG1DM), VP101(hG4WT) and bevacizumab.

Example 3: Affinity Constant Assay of FcγRIIa_R131 to VP101(hG1WT) and VP101(hG1DM)

The Fc receptor FcγRIIa_R131 (also known as CD32a_R131), can bind to the Fc fragment of IgG antibodies and mediate ADCC effects.

The affinity constants of VP101(hG1WT) and VP101(hG1DM) to FcγRIIa_R131 were determined using a Fortebio Octet system in this experiment to evaluate the ADCC activity of each antibody.

The method for determining the affinity constants of VP101(hG1WT) and VP101(hG1DM) using a Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL FcγRIIa_R131 was immobilized on an NTA sensor for an immobilization time of 60 s. The sensor was equilibrated in a buffer of PBS, 0.02% Tween-20, 0.02% casein and 0.1% BSA (pH 7.4) for 600 s of blocking, and the binding of the immobilized FcγRIIa_R131 on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

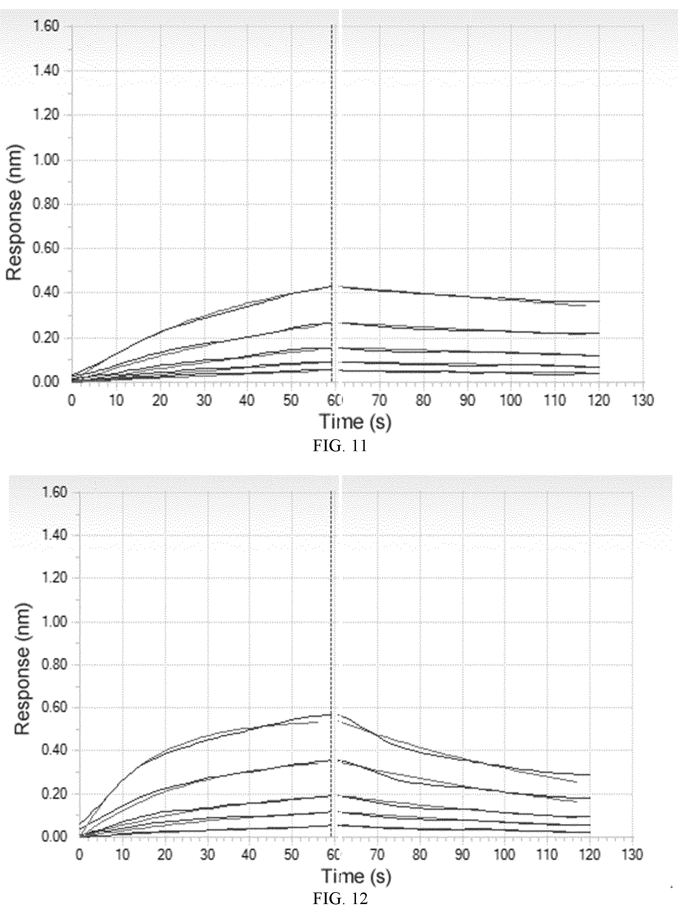

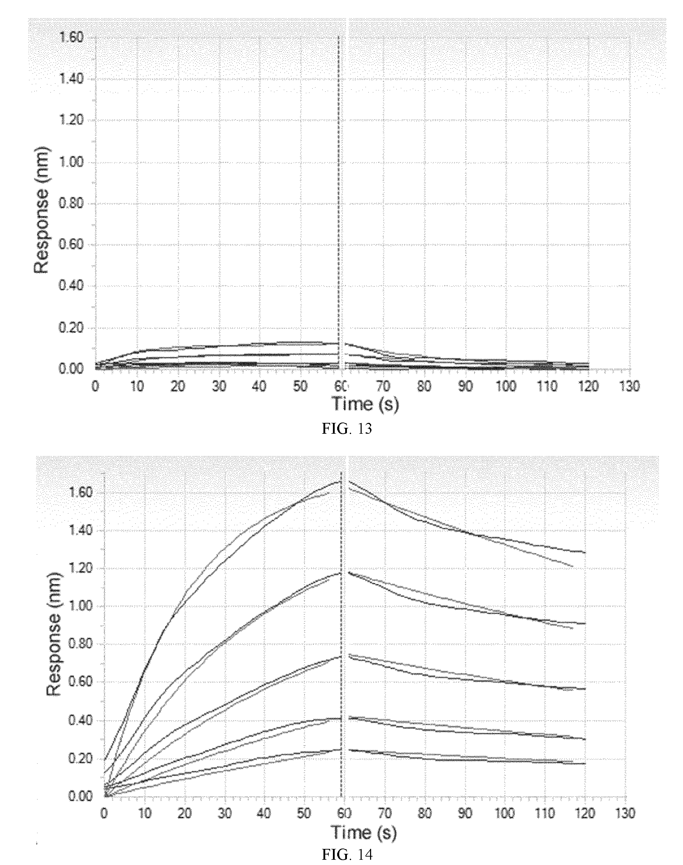

The affinity constants of FcγRIIa_R131 to VP101(hG1WT), VP101(hG4WT) and VP101(hG1DM) as well as control antibodies nivolumab and bevacizumab are shown in Table 3 and FIGs. 11-15.

Table 3: Kinetics for Binding of VP101(hG1WT) and VP101(hG1DM) and Isotypes Thereof to FcγRIIa_R131

| Antibody | KD (M) | kon (1/Ms) | SE (kon) | kdis (1/s) | SE (kdis) | Rmax (nm) |
|---|---|---|---|---|---|---|
| VP101(hG1DM) | 3.35E-08 | 1.20E+05 | 9.72E+03 | 4.03E-03 | 3.08E-04 | 0.57-0.69 |
| Bevacizumab | 5.16E-08 | 2.59E+05 | 1.72E+04 | 1.33E-02 | 4.52E-04 | 0.42-0.69 |
| Nivolumab | 6.93E-08 | 4.78E+05 | 1.09E+05 | 3.31E-02 | 2.54E-03 | 0.08-0.16 |
| VP101(hG1WT) | 2.42E-08 | 2.14E+05 | 1.30E+04 | 5.17E-03 | 3.28E-04 | 1.75-1.92 |
| VP101(hG4WT) | 3.57E-08 | 1.99E+05 | 1.23E+04 | 7.09E-03 | 3.40E-04 | 0.81-1.05 |
| N/A indicates that the antibody had no binding or an extremely weak binding signal to the antigen, and thus the results were not analyzed and no corresponding data was obtained. | | | | | | |

The results show that VP101(hG1WT) can bind to FcγRIIa_R131 with an affinity constant of 2.42E-08M; VP101(hG4WT) can bind to FcγRIIa_R131 with an affinity constant of 3.57E-08M; bevacizumab can bind to FcγRIIa_R131 with an affinity constant of 5.16E-08M; nivolumab can bind to FcγRIIa_R131 with an affinity constant of 6.93E-08M; VP101(hG1DM) can bind to FcγRIIa_R131 with an affinity constant of 3.35E-08M.

The results show that the antibodies bound to FcγRIIa_R131 with the following strong-to-weak affinities: VP101(hG1WT), VP101(hG1DM), VP101(hG4WT), bevacizumab and nivolumab.

Example 4: Affinity Constant Assay of FcγRIIb to VP101(hG1WT) and VP101(hG1DM)

The Fc receptor FcγRIIb (also known as CD32b), can bind to the Fc fragment of IgG antibodies and regulate functions of immune cells.

The affinity constants of VP101(hG1WT) and VP101(hG1DM) to FcγRIIb were determined using a Fortebio Octet system in this experiment to evaluate the binding ability of VP101(hG1WT) and VP101(hG1DM) to the Fc receptor.

The method for determining the affinity constants of VP101(hG1WT) and VP101(hG1DM) to FcγRIIb using a Fortebio Octet system is briefly described as follows: the immobilization dilution buffer was a solution of PBS, 0.02% Tween-20 and 0.1% BSA, pH 7.4, and the analyte dilution buffer was a solution of PBS, 0.02% Tween-20, 0.02% casein and 0.1% BSA, pH 7.4. 5 μg/mL hFCGR2B-his was immobilized on an NTA sensor for an immobilization time of 60 s. The sensor was equilibrated in a buffer of PBS, 0.02% Tween-20, 0.02% casein and 0.1% BSA (pH 7.4) for 600 s of blocking, and the binding of the immobilized hFCGR2B-his on the sensor to the antibodies at concentrations of 12.5-200 nM (serial two-fold dilution) was determined for 60 s. The antibodies were dissociated in the buffer for 60 s. The sensor was refreshed in 10 mM glycine pH 1.7 and 10 nM nickel sulfate. The detection temperature was 30 °C and the frequency was 0.6 Hz. The data were analyzed by 1:1 model fitting to obtain affinity constants.

Example 5: Affinity Constant Assay of FcγRIIIa_V158 to VP101(hG1WT) and VP101(hG1DM)

The Fc receptor FcγRIIIa_V158 (also known as CD16a_V158), can bind to the Fc fragment of IgG antibodies and mediate ADCC effects.

The affinity constants of VP101(hG1WT) and VP101(hG1DM) to FcγRIIIa_V158 were determined using a Fortebio Octet system in this experiment to evaluate the ADCC activity of each antibody.

The method for determining the affinity constant of the antibodies to FcγRIIIa_V158 by the Fortebio Octet system is briefly described as follows: the sample dilution buffer was a solution of PBS, 0.02% Tween-20 and 0.1% BSA, pH 7.4. 5 μg/mL FcγRIIIa_V158 was immobilized on an HIS1K sensor for 120 s. The sensor was equilibrated in a buffer for 60 s, and the binding of the immobilized hFcGR3A(V158)-his on the sensor to the antibodies at concentrations of 31.25-500 nM (serial two-fold dilution) was determined for 60 s. The antibodies were dissociated in the buffer for 60 s. The sensor was refreshed 4 times in 10 mM glycine pH 1.5, each for 5 s. The detection temperature was 30°C and the frequency was 0.3 Hz. The data were analyzed by 1: 1 model fitting to obtain affinity constants.

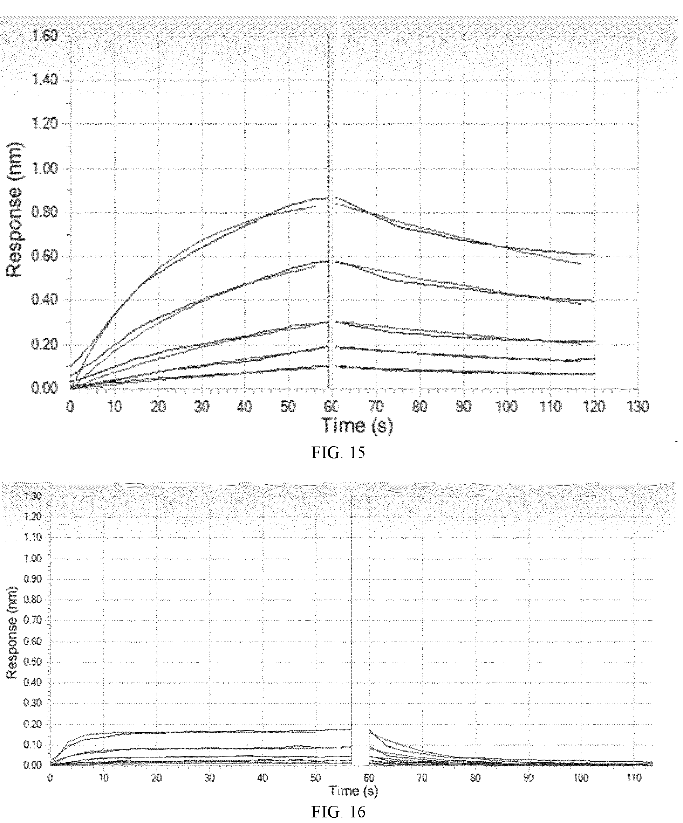

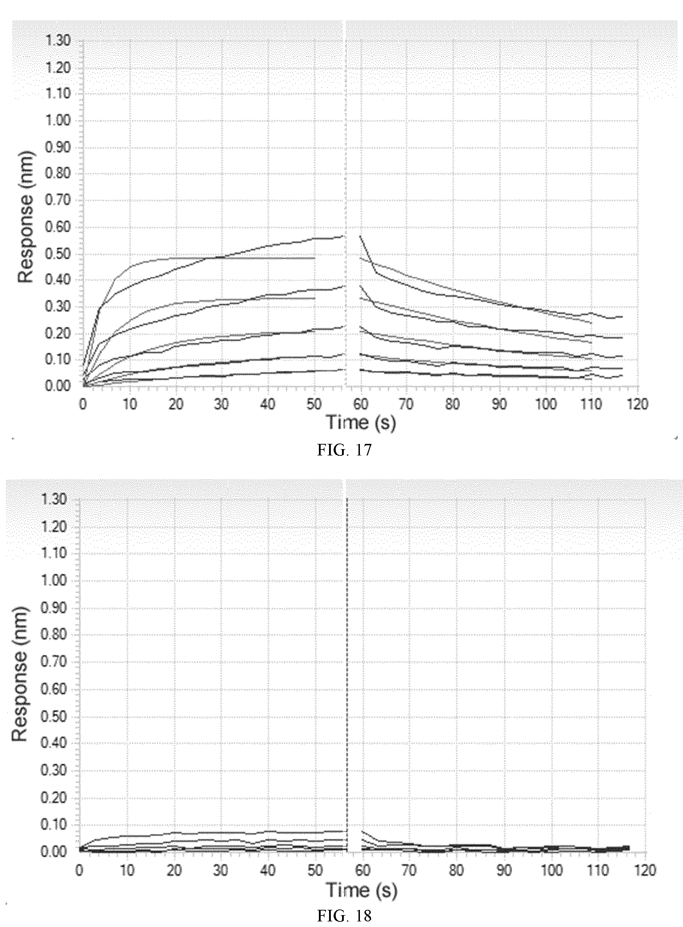

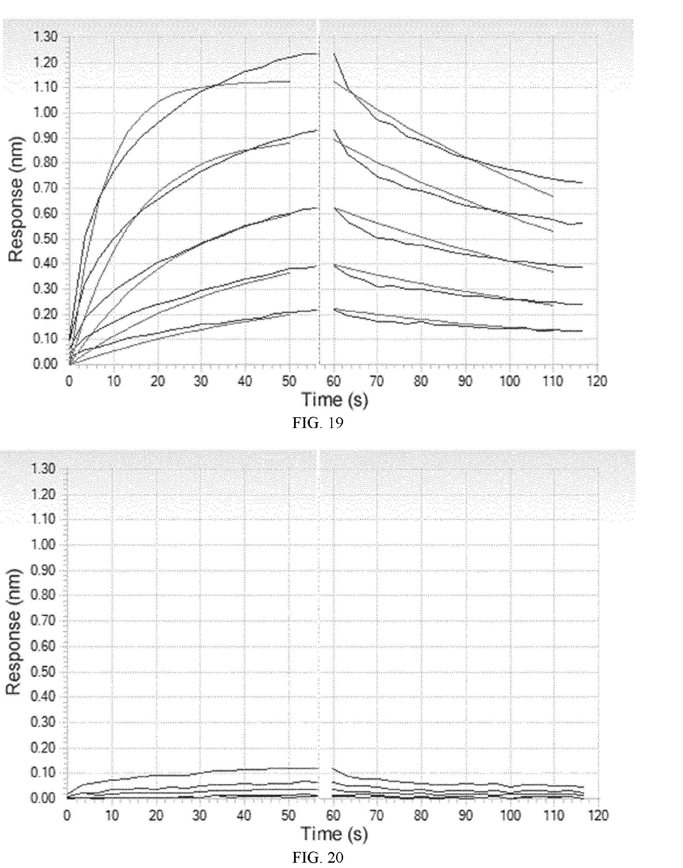

The affinity constants of FcγRIIIa_V158 to VP101(hG1WT), VP101(hG4WT) and VP101(hG1DM) as well as control antibodies nivolumab and bevacizumab are shown in Table 4 and FIGs. 16-20.

Table 4: Kinetics for Binding of VP101(hG1WT) and VP101(hG1DM) and Isotypes Thereof to FcγRIIIa_V158

| Antibody | $K_D$ (M) | kon (1/Ms) | SE (kon) | kdis (1/s) | SE (kdis) | Rmax (nm) |
|---|---|---|---|---|---|---|
| VP101(hG1DM) | 1.34E-07 | 6.05E+05 | 2.36E+05 | 8.11E-02 | 7.42E-03 | 0.07-0.21 |
| Bevacizumab | 2.76E-08 | 5.06E+05 | 1.14E+05 | 1.39E-02 | 1.41E-03 | 0.13-0.51 |
| Nivolumab | N/A | N/A | N/A | N/A | N/A | N/A |
| VP101(hG1WT) | 4.35E-08 | 2.39E+05 | 3.14E+04 | 1.04E-02 | 8.73E-04 | 0.80-1.22 |
| VP101(hG4WT) | N/A | N/A | N/A | N/A | N/A | N/A |
| N/A indicates that the antibody had no binding or an extremely weak binding signal to the antigen, and thus the results were not analyzed and no corresponding data was obtained. | | | | | | |

The results show that VP101(hG1WT) can bind to FcγRIIIa_V158 with an affinity constant of 4.35E-08M; VP101(hG1DM) can bind to FcγRIIIa_V158 with an affinity constant of 1.34E-07M; bevacizumab can bind to FcγRIIIa_V158 with an affinity constant of 2.76E-08M; while nivolumab and VP101(hG4WT) had no binding or an extremely low binding signal to FcγRIIIa_V158, and thus the results were not analyzed and no corresponding data were obtained. The results show that, except for no binding of nivolumab and VP101(hG4WT) to FcγRIIIa_V158, other antibodies bound to FcγRIIIa_V158 with the following strong-to-weak affinities: bevacizumab, VP101(hG1WT) and VP101(hG1DM).

Example 6: Affinity Constant Assay of FcγRIIIa_F158 to VP101(hG1WT) and VP101(hG1DM)

The Fc receptor FcγRIIIa_F158 (also known as CD16a_F158), can bind to the Fc fragment of IgG antibodies and mediate ADCC effects.

The affinity constants of VP101(hG1WT) and VP101(hG1DM) to FcγRIIIa_F158 were determined using a Fortebio Octet system in this experiment to evaluate the ADCC activity of each antibody.

The method for determining the affinity constants of VP101(hG1WT) and VP101(hG1DM) to FcγRIIIa_F158 using a Fortebio Octet system is briefly described as follows: the sample dilution buffer was a solution of PBS, 0.02% Tween-20 and 0.1% BSA, pH 7.4. 5 μg/mL FcγRIIIa_F158 was immobilized on an HIS1K sensor for 120 s. The sensor was equilibrated in a buffer for 60 s, and the binding of the immobilized hFcGR3A(F158)-his on the sensor to the antibodies at concentrations of 31.25-500 nM (serial two-fold dilution) was determined for 60 s. The antibody was dissociated in the buffer for 60 s. The sensor was refreshed 4 times in 10 mM glycine pH 1.5, each for 5 s. The detection temperature was 30 °C and the frequency was 0.3 Hz. The data were analyzed by 1:1 model fitting to obtain affinity constants.

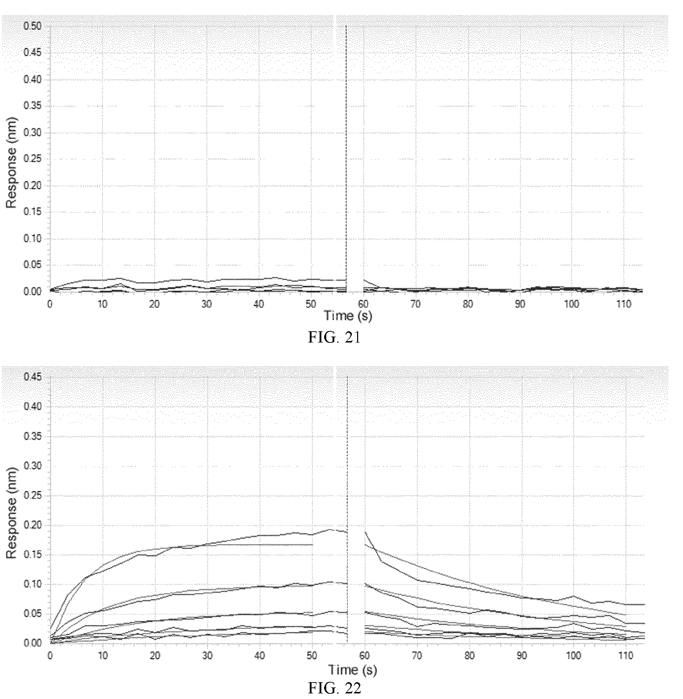

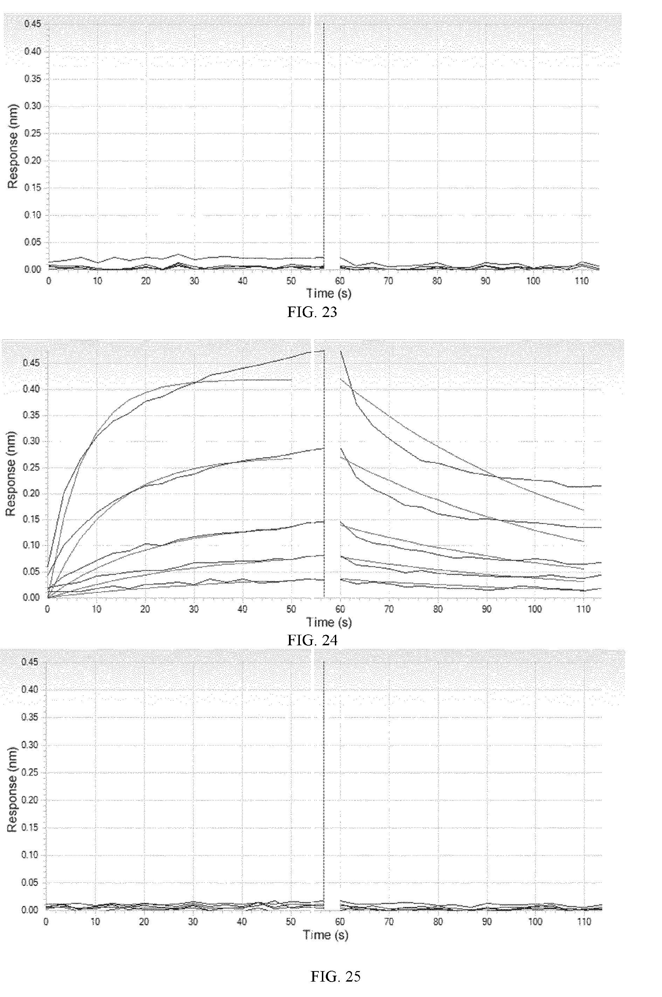

The affinity constants of FcγRIIIa_F158 to VP101(hG1WT), VP101(hG4WT) and VP101(hG1DM) as well as control antibodies nivolumab and bevacizumab are shown in Table 5 and FIGs. 21-25.

Table 5: Kinetics for Binding of VP101(hG1WT) and VP101(hG1DM) and Isotypes Thereof to FcγRIIIa_F158

| Antibody | $K_D$ (M) | kon (1/Ms) | SE (kon) | kdis (1/s) | SE (kdis) | Rmax (nm) |
|---|---|---|---|---|---|---|
| VP101(hG1DM) | N/A | N/A | N/A | N/A | N/A | N/A |
| Bevacizumab | 9.32E-08 | 2.64E+05 | 7.16E+04 | 2.46E-02 | 2.09E-03 | 0.08-0.20 |
| Nivolumab | N/A | N/A | N/A | N/A | N/A | N/A |

(continued)

| Antibody | $K_D$ (M) | kon (1/Ms) | SE (kon) | kdis (1/s) | SE (kdis) | Rmax (nm) |
|---|---|---|---|---|---|---|
| VP101(hG1WT) | 7.41E-08 | 2.47E+05 | 5.20E+04 | 1.83E-02 | 1.55E-03 | 0.15-0.48 |
| VP101(hG4WT) | N/A | N/A | N/A | N/A | N/A | N/A |

N/A indicates that the antibody had no binding or an extremely weak binding signal to the antigen, and thus the results were not analyzed and no corresponding data was obtained.

The results show that VP101(hG1WT) can bind to FcγRIIIa_F158 with an affinity constant of 7.41E-08M; bevacizumab can bind to FcγRIIIa_F158 with an affinity constant of 9.32E-08M; while nivolumab, VP101(hG4WT) and VP101(hG1DM) had no binding or an extremely low binding signal to FcγRIIIa_F158, and thus the results were not analyzed and no corresponding data were obtained.

The results show that, except for no binding of nivolumab, VP101(hG4WT) and VP101(HG1DM) to FcγRIIIa_F158, other antibodies bound to FcγRIIIa_F158 with the following strong-to-weak affinities: VP101(hG1WT) and bevacizumab.

Example 7: Affinity Constant Assay of C1q to VP101(hG1WT) and VP101(hG1DM)

Serum complement C1q can bind to the Fc fragment of IgG antibodies and mediate CDC effects. The binding capacity of a therapeutic monoclonal antibody to C1q will influence the safety and efficacy of the antibody.

The affinity constants of VP101(hG1WT) and VP101(hG1DM) to C1q were determined using a Fortebio Octet system in this experiment to evaluate the CDC activity of each antibody.

The method for determining the affinity constant of the antibodies to C1q using a Fortebio Octet system is briefly described as follows: the sample dilution buffer was a solution of PBS, 0.02% Tween-20 and 0.1% BSA, pH 7.4. 50 μg/mL of antibodies were immobilized on an FAB2G sensor at an immobilization height of about 2.0 nm. The sensor was equilibrated in a buffer for 60 s, and the binding of the immobilized antibodies on the sensor to C1q at concentrations of 0.625-10 nM (serial two-fold dilution) was determined for 60 s. The antigen and the antibodies were dissociated in the buffer for 60 s. The sensor was refreshed 4 times in 10 mM glycine pH 1.7, each for 5 s. The shaking speed of the sample plate was 1000 rpm, the temperature was 30 °C and the frequency was 0.6 Hz. The data were analyzed by 1: 1 model fitting to obtain affinity constants. The data acquisition software was Fortebio Data Acquisition 7.0, and the data analysis software was Fortebio Data Analysis 7.0.

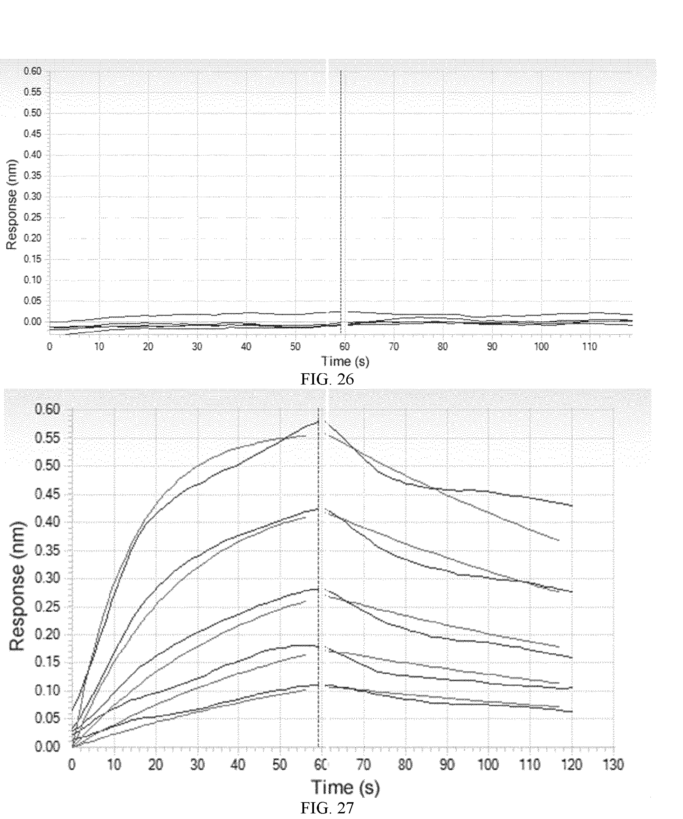

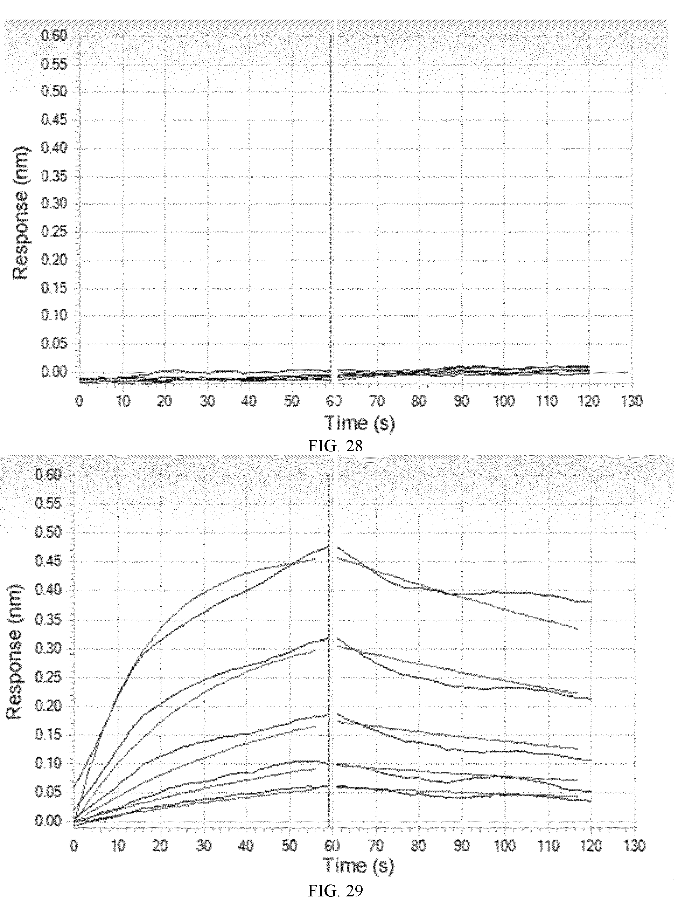

The affinity constants of C1q to VP101(hG1WT), VP101(hG4WT) and VP101(hG1DM) as well as control antibodies nivolumab and bevacizumab are shown in Table 6 and FIGs. 26-30.

Table 6: Kinetics for Binding of VP101(hG1WT) and VP101(hG1DM) and Isotypes Thereof to C1q

| Antibody | $K_D$ (M) | kon (1/Ms) | SE (kon) | kdis (1/s) | SE (kdis) | Rmax (nm) |
|---|---|---|---|---|---|---|
| VP101(hG1DM) | N/A | N/A | N/A | N/A | N/A | N/A |
| Bevacizumab | 1.14E-09 | 6.52E+06 | 5.64E+05 | 7.40E-03 | 5.58E-04 | 0.51-0.63 |
| Nivolumab | N/A | N/A | N/A | N/A | N/A | N/A |
| VP101(hG1WT) | 9.76E-10 | 5.73E+06 | 5.49E+05 | 5.59E-03 | 6.12E-04 | 0.32-0.51 |
| VP101(hG4WT) | N/A | N/A | N/A | N/A | N/A | N/A |

N/A indicates that the antibody had no binding or an extremely weak binding signal to the antigen, and thus the results were not analyzed and no corresponding data was obtained.

The results show that VP101(hG1WT) can bind to C1q with an affinity constant of 9.76E-10M; bevacizumab can bind to C1q with an affinity constant of 1.14E-09; while nivolumab, VP101(hG4WT) and VP101(hG1DM) had no binding or an extremely low binding signal to C1q, and thus the results were not analyzed and no corresponding data were obtained.

The results show that nivolumab, VP101(hG4WT) and VP101(hG1DM) have no binding to C1q, and VP101(hG1WT) and bevacizumab bind to C1q with similar affinities.

Example 8: ADCC Activity Assay of VP101(hG1WT) and VP101(hG1DM) on CHO-K1-PD1 Cells Expressing PD-1 Antigen

In order to detect the ADCC effect of the antibodies VP101(hG1WT) and VP101(hG1DM) at the cellular level, the inventors constructed CHO-K1-PD1 cells expressing PD-1 antigen, and established a system of co-culture of normal human PBMC and target cells for detecting the ADCC activity of the antibodies at the cytological level.

The method for detecting the ADCC activity of VP101(hG1WT) and VP101(hG1DM) on CHO-K1-PD1 cells expressing PD-1 antigen is specified as follows:

In this experiment, a human PD-1 overexpression vector pCDH-CMV-PD1FL-Puro (pCDH-CMV-Puro purchased from Youbio) was firstly constructed, the expression vector was packaged by virus and then infected CHO-K1 cells, and a CHO-K1-PD1 stable cell line of the drug-resistant stable expression membrane PD-1 protein was obtained after dosing and screening with Puromycin (2 $\mu$g/mL). Normal human PBMCs were isolated according to the Ficoll peripheral blood mononuclear cell isolation instruction. The isolated PBMCs were resuspended in a 1640 complete medium and stained with trypan blue, cells were counted and the viability of cells was determined. The cells were incubated overnight in a saturated humidity incubator at 37 °C and 5% $CO_2$. The next day, CHO-K1-PD1 cells and PBMC were collected and centrifuged to remove the supernatant, and then the cell pellets were resuspended in RPMI-1640 (containing 1% BSA) (hereinafter referred to as assay medium), centrifuged and washed 2 times; the cells were counted and the viability of cells was determined, the concentration of the cells was adjusted to a proper range by using the assay medium, and the CHO-K1-PD1 cell suspension (30,000/well) was added into a 96-well plate according to the experimental design; 50 $\mu$L of antibody was added, mixed well, and pre-incubated for 1 h at room temperature; after pre-incubation, the cells were added with PBMC (900,000/50 $\mu$L/well), mixed well, and incubated for 4 h in a incubator at 37 °C and 5% $CO_2$. After 4 h, the 96-well plate was removed and centrifuged for 5 min at 250$\times$ g; 100 $\mu$L of the cell supernatant was carefully transferred to a new 96-well flat-bottom microplate (do not pipette the cell precipitate). 100 $\mu$L of freshly prepared reaction solution was added to each well according to the Cytotoxicity Detection Kit instruction. The cells were incubated for 30 min at room temperature in the dark. OD values at 490 nm and 650 nm were measured, wherein the OD values of each group = $OD_{490nm}$ - $OD_{650nm}$. ADCC activity was calculated for each group according to the formula ADCC(%) = (treatment group - negative control group)/(maximum LDH release in target cell - spontaneous LDH release in target cell) $\times$ 100%.

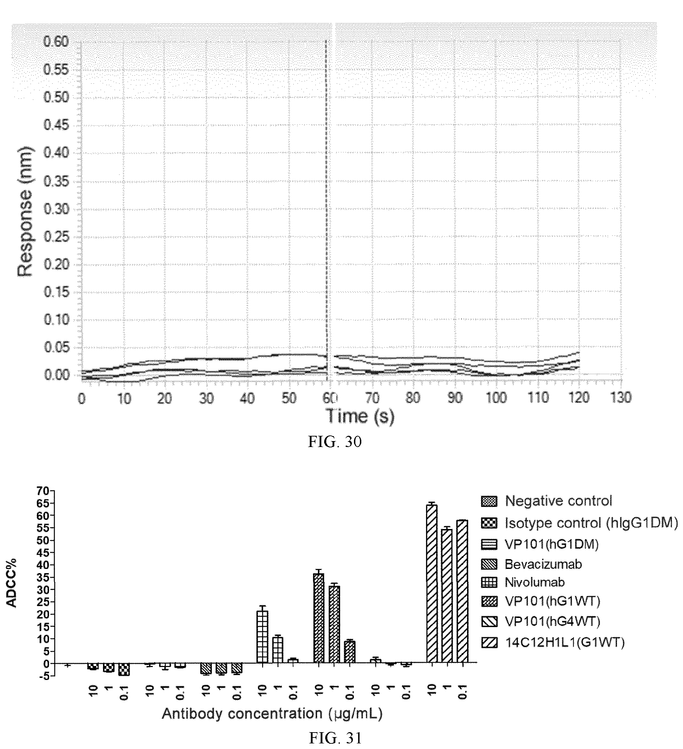

The detection results of the ADCC activity of VP101(hG1WT) and VP101(hG1DM) on CHO-K1-PD1 cells expressing the PD-1 antigen are expressed as ADCC%, and the results are shown in FIG. 31.

The results show that the positive control 14C12H1L1(G1WT) has significant ADCC activity in the mixed culture system of PBMC and CHO-K1-PD1, indicating that the ADCC system is normal. Compared to the isotype control antibody hIgG1DM, VP101(hG1WT) showed significant ADCC activity and exhibited dose-dependence, whereas VP101(G1DM) did not see ADCC activity. The results show that VP101(hG1DM) produced by mutation based on VP101(hG1WT) has no ADCC activity at the cytological level, and the ADCC effect is eliminated.

Example 9: CDC Activity Assay of VP101(hG1WT) and VP101(hG1DM) on CHO-K1-PD1 Cells Expressing PD-1 Antigen

In order to detect the CDC effect of the antibodies VP101(hG1WT) and VP101(hG1DM) at the cytological level, the inventors constructed CHO-K1-PD1 cells expressing PD-1 antigen (see Example 8 for the construction method), and established a system of co-culture of target cells and normal human complement serum for detecting the CDC activity of the antibodies at the cytological level.

The method for detecting the CDC activity of VP101(hG1WT) and VP101(hG1DM) on CHO-K1-PD1 cells expressing PD-1 antigen is specified as follows:

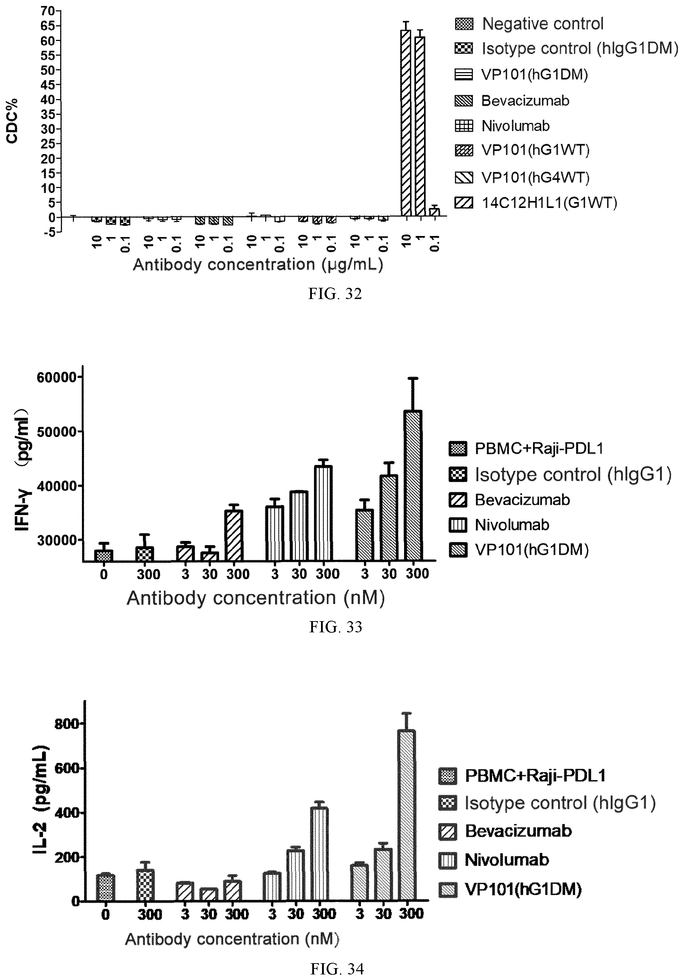

On the day of detection, CHO-K1-PD1 cells were collected by trypsinization and centrifuged at 170$\times$ g for 5 min; the cell pellets were resuspended in RPMI-1640 (containing 1% BSA) (hereinafter referred to as assay medium), repeatedly centrifuged and washed 2 times; the cells were counted and the viability of cells was determined, the concentration of the cells was adjusted to a proper range by using the assay medium, and the CHO-K1-PD1 cell suspension (30,000/well) was added into a 96-well plate according to the experimental design; 50 $\mu$L of antibody was added, mixed well, and pre-incubated for 10 min at room temperature; after pre-incubation, the cells were added with normal human complement serum (final concentration: 2%) at 50 $\mu$L/well, mixed well, and incubated for 4 h in an incubator at 37 °C and 5% $CO_2$. After 4 h, the cells were centrifuged for 5 min at 250$\times$ g; 100 $\mu$L of the cell supernatant was carefully transferred to a new 96-well flat-bottom plate (do not pipette the cell precipitate). 100 $\mu$L of freshly prepared reaction solution was added to each well according to the Cytotoxicity Detection Kit instruction. The cells were incubated for 30 min at room temperature in the dark. OD values at 490 nm and 650 nm were measured, wherein the OD values of each group = $OD_{490nm}$ - $OD_{650nm}$. CDC activity was calculated for each group according to the formula CDC(%) = (treatment group - negative control group)/(-maximum LDH release in target cell - spontaneous LDH release in target cell) $\times$ 100%. The detection results of the CDC activity of VP101(hG1WT) and VP101(hG1DM) on CHO-K1-PD1 cells expressing the PD-1 antigen are expressed as CDC%, and the results are shown in FIG. 32.

The results show that CDC% of the positive control antibody 14C12H1L1(G1WT) had significant difference from that of the isotype control antibody hIgG1DM group in the mixed culture system of normal human complement serum and CHO-K1-PD1, indicating that the CDC detection system is normal. At equivalent dose levels, there was no significant difference in CDC% for both VP101(hG1WT) and VP101(hG1DM) compared to the isotype control.

Example 10: Pharmacodynamic Activities of VP101(hG1DM) in Mixed Culture System (MLR) of Peripheral Blood Mononuclear Cells and Raji-PDL1 Cells

In this experiment, a human PD-L1 overexpression vector plenti6.3-PD-L1-BSD (plenti6.3-BSD purchased from invitrogen) was firstly constructed, the expression vector was packaged by virus and then infected Raji cells, and a Raji-PDL1 stable cell line for stably expressing membrane PD-L1 protein was obtained after dosing and screening with BSD (10 $\mu$g/mL). Normal human PBMCs were isolated according to the Ficoll peripheral blood mononuclear cell isolation instruction, and the isolated PBMCs were resuspended in a 1640 complete medium, counted and frozen. The PBMCs were recovered, added with SEB (Staphylococcus aureus enterotoxin B) and stimulated to culture for two days. Two days later, Raji-PDL1 cells in the logarithmic phase were collected, added with mitomycin C (Sigma, working concentration was 25 $\mu$g/mL) and incubated in an incubator for 60 min, and the mitomycin C-treated Raji-PDL1 cells were centrifuged and washed; PBMCs stimulated with SEB for two days were collected and washed; these cells were mixed and cultured according to a proportion of 1:1 of the cell number under the condition of the presence or absence of antibodies. 3 days later, cell supernatant was collected by centrifugation, and IL-2 and IFN-$\gamma$ concentrations in the supernatant were detected by ELISA.

The results of secretion of IFN-$\gamma$ are shown in FIG. 33. The results show that VP101(hG1DM) can effectively promote the secretion of IFN-$\gamma$, and its activity is significantly superior to that of nivolumab.

The results of secretion of IL-2 are shown in FIG. 34. The results show that VP101(hG1DM) can effectively promote the secretion of IL-2 in a dose-dependent relationship, and its activity is significantly superior to that of nivolumab.

Example 11: No Antibody-Dependent Phagocytic Activity of VP101(hG1DM)on PD-1-Positive Cells

Antibody dependent cellular phagoxytosis (ADCP) means that the Fc fragment of an antibody bound to a cell surface antigen binds to the Fc receptor of a phagocytically active cell (such as a macrophage), and thus mediates phagocytosis of the antibody-bound cells. For immune checkpoint inhibitor antibodies, such as the PD-1 antibody, the presence of ADCP activity will cause damage to immune cells expressing PD-1 that exert an anti-tumor killing effect, thereby affecting their anti-tumor activity.

In this experiment, murine macrophages were taken as effector cells, PD-1 overexpression CHO-K1-PD1 cell lines (see Example 8 for the construction method) were taken as target cells. The ADCP effect mediated by the cells was detected. In this experiment, flow cytometry was adopted to detect the ADCP activity of VP101(hG1DM) on cells expressing PD-1, and the results show that VP101(hG1DM) had no ADCP activity, whereas the marketed antibody nivolumab for the same target had significant ADCP activity. The method is specifically as follows:

The femoral bone marrow of C57 mice (purchased from Guangdong Medical Laboratory Animal Center) was first aseptically collected and lysed by erythrocyte lysis buffer on ice for 5 min. The lysis was terminated with DMEM complete medium (containing 10% FBS), and the lysate was centrifuged at 1000 rpm and washed twice. The cell pellet was resuspended in 10 mL of DMEM complete medium and M-CSF were added at a working concentration of 100 ng/mL. The cells were cultured for 7 days at 37 °C and 5% $CO_2$ in a cell culture chamber for induction. Half of the medium was exchanged and M-CSF was added on days 3 and 5. The induction of cells was completed on day 7. The cells were digested with 0.25% trypsin. Macrophages were collected, and centrifuged at 750× g for 5 min. The supernatant was removed and the cells were suspended in DMEM complete medium (containing 10% FBS) and counted. The cells were adjusted to a proper density and filled into a 96-well conical bottom plate for later use.

CHO-K1-PD1 cells were collected by conventional methods, centrifuged at 170× g for 5 min, resuspended and counted, and the viability was determined. The cells were washed once with PBS. Carboxyfluorescein diacetate succinimidyl ester (CFSE) was diluted to 2.5 $\mu$M with PBS, and the cells were resuspended with an appropriate amount of diluted CFSE (staining density: 10,000,000 cells/mL) and incubated in an incubator for 20 min. 6 mL of DMEM complete medium (containing 10% FBS) was added to stop staining. The cells were centrifuged at 170× g for 5 min, and the supernatant was removed; 1 mL of DMEM complete medium was added. The cells were incubated in an incubator for 10 min. The antibodies were diluted with DMEM complete medium to 20 $\mu$g/mL, 2 $\mu$g/mL and 0.2 $\mu$g/mL (working concentrations were 10 $\mu$g/mL, 1 $\mu$g/mL and 0.1 $\mu$g/mL), and isotype control antibodies hIgG1DM and hIgG4 were designed. Fresh induced mature macrophages were collected and centrifuged at 750× g for 5 min, and the supernatant was removed; the cells were counted and transferred to a 96-well conical bottom plate, and centrifuged at 1000× g for 5 min, and the supernatant was removed; the cell density of CHO-K1-PD 1-CFSE was adjusted; the diluted antibodies and the target cells were mixed according to the experimental design according to a proportion of 50 µL: 50 µL into the 96-well conical bottom plate containing the macrophages. The cells were resuspended and mixed well, and incubated in an incubator at 37 °C for 2 h. 150 µL of normal-temperature 1% PBSA was added into each well and centrifuged at 1000× g for 5 min, and the supernatant was removed; the cells were washed once with 200 µL of PBSA; APC anti-mouse/human CD11b antibody (500-fold diluted with PBSA) was added to the corresponding samples at 100 µL/sample, mixed well and incubated on ice for 40 min. 150 µL of 1% PBSA was added into each well and centrifuged at 1000× g for 5 min, and the supernatant was removed; each well was washed once with 200 µL of PBSA. 200 µL of 1% PBSA was added into each well for resuspension followed by analysis using a Beckman flow cytometer.

Macrophages in the system were APC⁺ positive, and macrophages involved in phagocytosis were APC and CFSE double positive. The phagocytosis rate was determined as the ratio of the number of double positive cells to the number of APC positive cells, and the antibody-dependent ADCP activity was evaluated. The ADCP activity of each group, represented by P%, was calculated according to the following formulas:

$$P\% = \frac{\text{Number of macrophages involved in phagocytosis}}{\text{Total number of macrophages}} \times 100\%$$

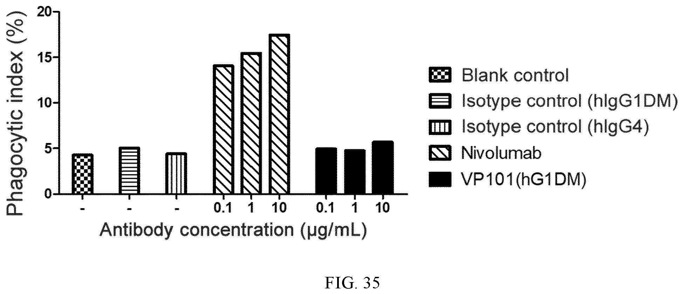

The results are shown in FIG. 35.

The results show that nivolumab had a significant ADCP effect in the macrophage + CHO-K1-PD1 system; the phagocytosis rate of VP101(hG1DM) was comparable to that of the isotype control antibody, indicating that VP101(hG1DM) had no ADCP effect. The results indicate that VP101(hG1DM) is likely to have a better anti-tumor effect.

**Claims**

1. A bispecific antibody, comprising:

(a) an immunoglobulin targeting VEGF-A, wherein the amino acid sequence of the heavy chain of the immunoglobulin is set forth in SEQ ID NO: 24, and the amino acid sequence of the light chain of the immunoglobulin is set forth in SEQ ID NO: 26; and
(b) two scFvs targeting PD-1; wherein, for each of the two scFvs, the amino acid sequence of the heavy chain variable region is set forth in SEQ ID NO: 9, and the amino acid sequence of the light chain variable region is set forth in SEQ ID NO: 17;

wherein:

(i) one scFv is attached via a linker to the C-terminus of one of the two heavy chains of the immunoglobulin; and the other scFv is attached via a linker to the C-terminus of the other of the two heavy chains of the immunoglobulin; and
(ii) for each of the two scFvs, the heavy chain variable region and the light chain variable region are attached via a linker;

and wherein the linkers of (i) and the linkers of (ii) are the same or different, and are independently selected from SEQ ID NO: 18 and SEQ ID NO: 19.

2. The bispecific antibody according to claim 1, wherein the amino acid sequences of the linkers of (i) and the amino acid sequences of the linkers of (ii) are set forth in SEQ ID NO: 18.

3. An isolated nucleic acid molecule, encoding the bispecific antibody according to claim 1 or 2.

4. A vector, comprising the isolated nucleic acid molecule according to claim 3.

5. A host cell, comprising the isolated nucleic acid molecule according to claim 3 or the vector according to claim 4.

6. A conjugate, comprising the bispecific antibody according to claim 1 or 2 and a conjugated moiety, wherein the conjugated moiety is a detectable label; optionally wherein the conjugated moiety is a radioisotope, a fluorescent substance, a luminescent substance, a colored substance, or an enzyme.

7. A kit, comprising the bispecific antibody according to claim 1 or 2 or the conjugate according to claim 6; optionally wherein the kit further comprises a second antibody capable of specifically recognizing the immunoglobulin or an antigen binding fragment thereof; optionally, the second antibody further comprises a detectable label, for example, a radioisotope, a fluorescent substance, a luminescent substance, a colored substance, or an enzyme.

8. A pharmaceutical composition, comprising the bispecific antibody according to claim 1 or 2, wherein, optionally, the pharmaceutical composition further comprises a pharmaceutically acceptable vector and/or excipient.

9. The bispecific antibody according to claim 1 or 2 or the pharmaceutical composition according to claim 8 for use in the treatment and/or prevention of a malignant tumor in a subject.

10. The bispecific antibody for use according to claim 9 or the pharmaceutical composition for use according to claim 9, wherein the malignant tumor is selected from colon cancer, rectal cancer, lung cancer, liver cancer, ovarian cancer, skin cancer, glioma, melanoma, lymphoma, renal tumor, prostate cancer, bladder cancer, gastrointestinal cancer, breast cancer, brain cancer, cervical cancer, esophageal cancer, microsatellite instability-high (MSI-H) and deficient mismatch repair (dMMR) cancer, urothelial cancer, mesothelioma, endometrial cancer, gastric adenocarcinoma, gastroesophageal junction adenocarcinoma and leukemia.

11. The bispecific antibody for use according to claim 10 or the pharmaceutical composition for use according to claim 10, wherein:

    the lung cancer is non-small cell lung cancer or small cell lung cancer;
    the liver cancer is hepatocellular carcinoma;
    the renal tumor is renal cell carcinoma;
    the breast cancer is triple negative breast cancer; or
    the urothelial cancer is bladder cancer.

12. The bispecific antibody for use according to claim 11 or the pharmaceutical composition for use according to claim 11, wherein the non-small cell lung cancer is EGFR and/or ALK sensitive mutant non-small cell lung cancer.

13. The bispecific antibody for use according to claim 11 or the pharmaceutical composition for use according to claim 11, wherein the breast cancer is triple negative breast cancer.

14. The bispecific antibody for use according to any one of claims 9-13 or the pharmaceutical composition for use according to any one of claims 9-13, wherein the bispecific antibody or pharmaceutical composition is administered by intravenous infusion or injection to the subject.

15. The bispecific antibody for use according to any one of claims 9-14 or the pharmaceutical composition for use according to any one of claims 9-14, wherein the bispecific antibody or pharmaceutical composition is used in combination with a tumor chemotherapeutic drug, optionally wherein the tumor chemotherapeutic drug is administered prior to, concurrently with, or subsequent to the administration of the bispecific antibody or pharmaceutical composition.

16. The bispecific antibody for use according to any one of claims 9-15 or the pharmaceutical composition for use according to any one of claims 9-15, wherein the bispecific antibody or pharmaceutical composition is administered to the subject as a single administration, multiple administrations over a period of time, or, by reducing or increasing the dose proportionally with the emergency degree of the treatment.

17. The bispecific antibody for use according to any one of claims 9-16 or the pharmaceutical composition for use according to any one of claims 9-16, wherein the bispecific antibody or pharmaceutical composition is administered to the subject at a dose of 0.02-50 mg/kg, 0.1-50 mg/kg, 0.1-25 mg/kg, or 1-10 mg/kg.

**Patentansprüche**

1. Bispezifischer Antikörper, umfassend:

    (a) ein auf Immunglobulin zielendes VEGF-A, wobei die Aminosäuresequenz der schweren Kette des Immunglobulins in SEQ ID NO: 24 dargelegt ist und die Aminosäuresequenz der leichten Kette des Immunglobulins in SEQ ID NO: 26 dargelegt ist; und

(b) zwei auf scFvs zielende PD-1; wobei für jedes der zwei scFvs die Aminosäuresequenz der variablen Region der schweren Kette in SEQ ID NO: 9 dargelegt ist, und die Aminosäuresequenz der variablen Region der leichten Kette in SEQ ID NO: 17 dargelegt ist;

wobei:

(i) ein scFv über einen Linker mit dem C-Terminus einer der zwei schweren Ketten des Immunglobins verbunden ist; und das andere scFv über einen Linker mit dem C-Terminus der anderen der zwei schweren Ketten des Immunglobins verbunden ist; und
(ii) für jedes der zwei scFvs die variable Region der schweren Kette und die variable Region der leichten Kette über einen Linker verbunden sind;

und wobei die Linker von (i) und die Linker von (ii) gleich oder unterschiedlich sind und unabhängig voneinander aus SEQ ID NO: 18 und SEQ ID NO: 19 ausgewählt sind.

2. Bispezifischer Antikörper nach Anspruch 1, wobei die Aminosäuresequenzen der Linker von (i) und die Aminosäuresequenzen der Linker von (ii) in SEQ **ID** NO: 18 dargelegt sind.

3. Isoliertes Nukleinsäuremolekül, das den bispezifischen Antikörper nach Anspruch 1 oder 2 codiert.

4. Vektor, umfassend das isolierte Nukleinsäuremolekül nach Anspruch 3.

5. Wirtszelle, umfassend das isolierte Nukleinsäuremolekül nach Anspruch 3 oder den Vektor nach Anspruch 4.

6. Konjugat, umfassend den bispezifischen Antikörper nach Anspruch 1 oder 2 und eine konjugierte Einheit, wobei die konjugierte Einheit eine erfassbare Markierung ist; wobei die konjugierte Einheit wahlweise ein Radioisotop, eine fluoreszierende Substanz, eine lumineszierende Substanz, eine gefärbte Substanz oder ein Enzym ist.

7. Kit, umfassend den bispezifischen Antikörper nach Anspruch 1 oder 2 oder das Konjugat nach Anspruch 6; wobei wahlweise das Kit einen zweiten Antikörper umfasst, der imstande ist, spezifisch das Immunglobin oder ein Antigenbindungsfragment davon zu erkennen; wahlweise der zweite Antikörper weiter eine erfassbare Markierung, beispielweise ein Radioisotop, eine fluoreszierende Substanz, eine lumineszierende Substanz, eine gefärbte Substanz oder ein Enzym, umfasst.

8. Pharmazeutische Zusammensetzung, umfassend den bispezifischen Antikörper nach Anspruch 1 oder 2, wobei die pharmazeutische Zusammensetzung wahlweise weiter einen pharmazeutisch akzeptablen Vektor und/oder Hilfsstoff umfasst.

9. Bispezifischer Antikörper nach Anspruch 1 oder 2 oder pharmazeutische Zusammensetzung nach Anspruch 8 zur Verwendung bei der Behandlung und/oder Vorbeugung eines bösartigen Tumors in einer Person.

10. Bispezifischer Antikörper nach Anspruch 9 oder pharmazeutische Zusammensetzung nach Anspruch 9, wobei der bösartige Tumor aus Darmkrebs, Rektumkrebs, Lungenkrebs, Leberkrebs, Eierstockkrebs, Hautkrebs, Gliom, Melanom, Lymphom, Nierentumor, Prostatakrebs, Blasenkrebs, Magen-Darm-Krebs, Brustkrebs, Hirntumor, Gebärmutterhalskrebs, Speiseröhrenkrebs, Krebs mit hoher Mikrosatelliteninstabilität (MSI-H) und defizienter Mismatch-Reparatur (dMMR), Urothelkarzinom, Mesotheliom, Endometriumkrebs, Magenadenokarzinom, Adenokarzinom des gastroösophagealen Übergangs und Leukämie ausgewählt ist.

11. Bispezifischer Antikörper nach Anspruch 10 oder pharmazeutische Zusammensetzung nach Anspruch 10, wobei:

der Lungenkrebs ein nicht-kleinzelliger Lungenkrebs oder ein kleinzelliger Lungenkrebs ist;
der Leberkrebs ein hepatozelluläres Karzinom ist;
der Nierentumor ein Nierenzellenkarzinom ist;
der Brustkrebs ein dreifach negativer Brustkrebs ist; oder
das Urothelialkarzinom Blasenkrebs ist.

**12.** Bispezifischer Antikörper nach Anspruch 11 oder pharmazeutische Zusammensetzung nach Anspruch 11, wobei der nicht-kleinzellige Lungenkrebs EGFR- und/oder ALK-sensitiver mutanter nicht-kleinzellige Lungenkrebs ist.

**13.** Bispezifischer Antikörper nach Anspruch 11 oder pharmazeutische Zusammensetzung nach Anspruch 11, wobei der Brustkrebs dreifach negative Brustkrebs ist.

**14.** Bispezifischer Antikörper nach einem der Ansprüche 9-13 oder pharmazeutische Zusammensetzung nach einem der Ansprüche 9-13, wobei der bispezifische Antikörper oder die pharmazeutische Zusammensetzung der Person durch intravenöse Infusion oder Injektion verabreicht wird.

**15.** Bispezifischer Antikörper nach einem der Ansprüche 9-14 oder pharmazeutische Zusammensetzung nach einem der Ansprüche 9-14, wobei der bispezifische Antikörper oder die pharmazeutische Zusammensetzung in Kombination mit einem chemotherapeutischen Medikament gegen Tumoren verwendet wird, wobei das chemotherapeutische Medikament gegen Tumoren vor, gleichzeitig oder nach der Verabreichung des bispezifischen Antikörpers oder der pharmazeutischen Zusammensetzung verabreicht wird.

**16.** Bispezifischer Antikörper nach einem der Ansprüche 9-15 oder pharmazeutische Zusammensetzung nach einem der Ansprüche 9-15, wobei der bispezifische Antikörper oder die pharmazeutische Zusammensetzung der Person als eine einzige Verabreichung, mehrfache Verabreichung über einen Zeitraum oder durch Verringern oder Erhöhen der Dosierung proportional zum Dringlichkeitsgrad der Behandlung verabreicht wird.

**17.** Bispezifischer Antikörper nach einem der Ansprüche 9-16 oder pharmazeutische Zusammensetzung nach einem der Ansprüche 9-16, wobei der bispezifische Antikörper oder die pharmazeutische Zusammensetzung der Person in einer Dosierung von 0,02-50 mg/kg, 0,1-50 mg/kg, 0,1-25 mg/kg oder 1-10 mg/kg verabreicht wird.

**Revendications**

**1.** Anticorps bispécifique, comprenant :

(a) une immunoglobuline ciblant VEGF-A, dans lequel la séquence d'acides aminés de la chaîne lourde de l'immunoglobuline est définie dans SEQ **ID** NO : 24, et la séquence d'acides aminés de la chaîne légère de l'immunoglobuline est définie dans SEQ ID NO : 26 ; et
(b) deux scFv ciblant PD-1 ; dans lequel, pour chacun des deux scFv, la séquence d'acides aminés de la région variable de chaîne lourde est définie dans SEQ ID NO : 9, et la séquence d'acides aminés de la région variable de chaîne légère est définie dans SEQ ID NO : 17 ;

dans lequel :

(i) un scFv est fixé par l'intermédiaire d'un liant à la terminaison C de l'une des deux chaînes lourdes de l'immunoglobuline ; et l'autre scFv est fixé par l'intermédiaire d'un liant à la terminaison C de l'autre des deux chaînes lourdes de l'immunoglobuline ; et
(ii) pour chacun des deux scFv, la région variable de chaîne lourde et la région variable de chaîne légère sont fixées par l'intermédiaire d'un liant ;
et dans lequel les liants de (i) et les liants de (ii) sont identiques ou différents,
et sont indépendamment choisis parmi SEQ ID NO : 18 et SEQ ID NO : 19.

**2.** Anticorps bispécifique selon la revendication 1, dans lequel les séquences d'acides aminés des liants de (i) et les séquences d'acides aminés des liants de (ii) sont définies dans SEQ ID NO : 18.

**3.** Molécule d'acide nucléique isolée, codant l'anticorps bispécifique selon la revendication 1 ou la revendication 2.

**4.** Vecteur, comprenant la molécule d'acide nucléique isolée selon la revendication 3.

**5.** Cellule hôte, comprenant la molécule d'acide nucléique isolée selon la revendication 3 ou le vecteur selon la revendication 4.

**6.** Conjugué, comprenant l'anticorps bispécifique selon la revendication 1 ou la revendication 2 et un fragment conjugué, dans lequel le fragment conjugué est un marqueur détectable ; facultativement dans lequel le fragment conjugué est un radioisotope, une substance fluorescente, une substance luminescente, une substance colorée, ou une enzyme.

7. Kit, comprenant l'anticorps bispécifique selon la revendication 1 ou la revendication 2 ou le conjugué selon la revendication 6 ; facultativement dans lequel le kit comprend en outre un second anticorps en mesure de reconnaître spécifiquement l'immunoglobuline ou un fragment de liaison à l'antigène de celle-ci ; facultativement, le second anticorps comprend en outre un marqueur détectable, par exemple, un radioisotope, une substance fluorescente, une substance luminescente, une substance colorée, ou une enzyme.

8. Composition pharmaceutique, comprenant l'anticorps bispécifique selon la revendication 1 ou la revendication 2, dans laquelle, facultativement, la composition pharmaceutique comprend en outre un vecteur et/ou un excipient pharmaceutiquement acceptable.

9. Anticorps bispécifique selon la revendication 1 ou la revendication 2 ou composition pharmaceutique selon la revendication 8 destiné(e) à être utilisé(e) dans le traitement et/ou la prévention d'une tumeur maligne chez un sujet.

10. Anticorps bispécifique destiné à être utilisé selon la revendication 9 ou composition pharmaceutique destinée à être utilisée selon la revendication 9, dans lequel ou laquelle la tumeur maligne est choisie parmi cancer du côlon, cancer du rectum, cancer du poumon, cancer du foie, cancer de l'ovaire, cancer de la peau, gliome, mélanome, lymphome, tumeur rénale, cancer de la prostate, cancer de la vessie, cancer gastro-intestinal, cancer du sein, cancer du cerveau, cancer du col de l'utérus, cancer oesophagien, cancer à instabilité microsatellitaire élevée (MSI-H) et cancer lié à un déficit de réparation des mésappariements (dMMR), cancer urothélial, mésothéliome, cancer de l'endomètre, adénocarcinome gastrique, adénocarcinome de la jonction gastro-œsophagienne et leucémie.

11. Anticorps bispécifique destiné à être utilisé selon la revendication 10 ou composition pharmaceutique destinée à être utilisée selon la revendication 10, dans lequel ou laquelle :

le cancer du poumon est un cancer du poumon non à petites cellules ou un cancer du poumon à petites cellules ;
le cancer du foie est un carcinome hépatocellulaire ;
la tumeur rénale est un carcinome à cellules rénales ;
le cancer du sein est un cancer du sein triple négatif ; ou
le cancer urothélial est un cancer de la vessie.

12. Anticorps bispécifique destiné à être utilisé selon la revendication 11 ou composition pharmaceutique destinée à être utilisée selon la revendication 11, dans lequel ou laquelle le cancer du poumon non à petites cellules est un cancer du poumon non à petites cellules mutant sensible à l'EGFR et/ou à l'ALK.

13. Anticorps bispécifique destiné à être utilisé selon la revendication 11 ou composition pharmaceutique destinée à être utilisée selon la revendication 11, dans lequel ou laquelle le cancer du sein est un cancer du sein triple négatif.

14. Anticorps bispécifique destiné à être utilisé selon l'une quelconque des revendications 9 à 13 ou composition pharmaceutique destinée à être utilisée selon l'une quelconque des revendications 9 à 13, dans lequel ou laquelle l'anticorps bispécifique ou la composition pharmaceutique est administré(e) par perfusion ou injection au sujet.

15. Anticorps bispécifique destiné à être utilisé selon l'une quelconque des revendications 9 à 14 ou composition pharmaceutique destinée à être utilisée selon l'une quelconque des revendications 9 à 14, dans lequel ou laquelle l'anticorps bispécifique ou la composition pharmaceutique est utilisé(e) en combinaison d'un médicament chimiothérapeutique pour tumeur, facultativement dans lequel ou laquelle le médicament chimiothérapeutique pour tumeur est administré avant, simultanément à, ou après l'administration de l'anticorps bispécifique ou de la composition pharmaceutique.

16. Anticorps bispécifique destiné à être utilisé selon l'une quelconque des revendications 9 à 15 ou composition pharmaceutique destinée à être utilisée selon l'une quelconque des revendications 9 à 15, dans lequel ou laquelle l'anticorps bispécifique ou la composition pharmaceutique est administré(e) au sujet sous la forme d'une administration unique, de multiples administrations sur une période de temps, ou, en réduisant ou augmentant la dose proportionnellement au niveau d'urgence du traitement.

17. Anticorps bispécifique destiné à être utilisé selon l'une quelconque des revendications 9 à 16 ou composition pharmaceutique destinée à être utilisée selon l'une quelconque des revendications 9 à 16, dans lequel ou laquelle l'anticorps bispécifique ou la composition pharmaceutique est administré(e) au sujet à une dose comprise entre 0,02 et 50 mg/kg, entre 0,1 et 50 mg/kg, entre 0,1 et 25 mg/kg, ou entre 1 et 10 mg/kg.

EP 4 067 387 B1

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

FIG. 34

FIG. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 109053895 A
- US 5747654 A
- US 4816567 A
- CN 1259962 A
- CN 106967172 A

### Non-patent literature cited in the description

- **MA LI**. *Chinese Journal of Birth Health and Heredity*, 2016, vol. 24 (5), 146-148
- **ROSKOSKI R JR. et al.** *Crit Rev Oncol Hematol*, 2007, vol. 62 (3), 179-213
- **TAKAHASHI T et al.** *Oncogene*, 1999, vol. 18 (13), 2221-2230
- **DONG HONGCHAO et al.** *Journal of Modern Oncology*, September 2014, vol. 22 (9), 2231-3
- **HOMET M. B.** ; **PARISI G. et al.** Anti-PD-1 Therapy in Melanoma. *Semin Oncol.*, June 2015, vol. 42 (3), 466-473
- **HELD SA** ; **HEINE A et al.** Advances in immunotherapy of chronic myeloid leukemia CML. *Curr Cancer Drug Targets.*, September 2013, vol. 13 (7), 768-74
- **JOYCE F. LIU et al.** *JAMA Oncol.*, 2019, vol. 5 (12), 1731-1738
- **MANEGOLD C et al.** *J Thorac Oncol.*, February 2017, vol. 12 (2), 194-207
- **DUDEK AZ et al.** *J Clin Oncol.*, 2018, vol. 36
- **STEIN S et al.** *J Clin Oncol*, 2018, vol. 36 (15), 4074
- **BENDELL JC et al.** Safety and efficacy of MPDL3280A (anti-PDL1) in combination with bevacizumab (bev) and/or FOLFOX in patients (pts) with metastatic colorectal cancer (mCRC). *Presented at: American Society of Clinical Oncology; May 29-June 2, 2015; Chicago, IL*, 29 May 2015
- **HOCHSTER HS et al.** Efficacy and safety of atezolizumab (atezo) and bevacizumab (bev) in a phase Ib study of microsatellite instability (MSI)-high metastatic colorectal cancer (mCRC). *Presented at: American Society of Clinical Oncology Gastrointestinal Cancers Symposium; January 19-21, 2017; San Francisco, CA.*
- **YUKINORI OZAKI et al.** A multicenter phase II study evaluating the efficacy of nivolumab plus paclitaxel plus bevacizumab triple-combination therapy as a first-line treatment in patients with HER2-negative metastatic breast cancer: WJOG9917B NEWBEAT trial. *Proceedings of the 2019 San Antonio Breast Cancer Symposium; 2019 Dec 10-14; San Antonio, TX. Philadelphia (PA): AACR; Cancer Res*, vol. 80 (4)
- **HERBST RS et al.** *Lancet Oncol.*, 2019, vol. 20 (8), 1109-1123
- **KRISHNANSU S. et al.** *N Engl J Med.*, 2014, vol. 370, 734-743
- **ANTONARAKIS ES et al.** *J Clin Oncol.*, 10 February 2020, vol. 38 (5), 395-405
- **JOAQUIM BELLMUNT. et al.** *N Engl J Med.*, 2017, vol. 376, 1015-1026
- **KATO K et al.** *Lancet Oncol.*, 2019, vol. 20 (11), 1506-17
- **SCHERPEREEL A et al.** *Lancet Oncol.*, 2019, vol. 20 (2), 239-253
- **MILLER D** ; **KONTERMANN RE**. Bispecific antibodies for cancer immunotherapy: current perspectives. *BioDrugs*, 2010, vol. 24, 89-98
- **COLOMA M. J.** ; **MORRISON S. L.** Design and production of novel tetravalent bispecific antibodies.. *Nat Biotechnol.*, 1997, vol. 15, 159-163
- **MILLER B. R.** ; **DEMAREST S. J. et al.** Stability engineering of scFvs for the development of bispecific and multivalent antibodies. *Protein Eng Des Sel*, 2010, vol. 23, 549-57
- **FITZGERALD J** ; **LUGOVSKOY A**. Rational engineering of antibody therapeutics targeting multiple oncogene pathways. *MAbs*, 2011, vol. 3, 299-309
- **HOGARTH PM** ; **PIETERSZ GA**. *NATURE REVIEWS DRUG DISCOVERY*, 2012, vol. 11 (4), 311-331
- **RAJAGOPAL**. *Prot. Engin.*, 1997, vol. 10, 1453-1459
- **REITER**. *Nat. Biotechnol.*, 1996, vol. 14, 1239-1245

- **REITER**. *Protein Engineering*, 1995, vol. 8, 1323-1331
- **WEBBER**. *Molecular Immunology*, 1995, vol. 32, 249-258
- **REITER**. *Immunity*, 1995, vol. 2, 281-287
- **REITER**. *JBC*, 1994, vol. 269, 18327-18331
- **REITER**. *Inter. J. of Cancer*, 1994, vol. 58, 142-149
- **REITER et al.** *Cancer Res.*, 1994, vol. 54, 2714-2718
- **KABAT et al.** Bethesda M.d., Sequences of Proteins of Immunological Interest. 1991, vol. 1-3, 91-3242
- **EHRENMANN, FRANCOIS ; QUENTIN KAAS ; MARIE-PAULE LEFRANC**. IMGT/3Dstructure-DB and IMGT/DomainGapAlign: a database and a tool for immunoglobulins or antibodies, T cell receptors, MHC, IgSF and MhcSF. *Nucleic acids research*, 2009, vol. 38 (1), D301-D307
- **KABAT**. Sequences of Proteins of Immunological Interest. National Institutes of Health, 1987
- **CHOTHIA ; LESK**. *J. Mol. Biol.*, 1987, vol. 196, 901-917
- **CHOTHIA et al.** *Nature*, 1989, vol. 342, 878-883
- Fundamental Immunology. Raven Press, 1989
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol.*, 2001
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **KOHLER et al.** *Nature*, 1975, vol. 256, 495
- **MORRISON et al.** *Proc. Natl. Acad. Sci. USA*, 1984, vol. 81, 6851-6855
- **JONES et al.** *Nature*, 1986, vol. 321, 522-525
- **REICHMANN et al.** *Nature*, 1988, vol. 332, 323-329
- **PRESTA**. *Curr. Op. Struct. Biol.*, 1992, vol. 2, 593-596
- **CLARK**. *Immunol. Today*, 2000, vol. 21, 397-402
- Epitope Mapping Protocols in Methods in Molecular Biology. 1996, vol. 66
- Remington's Pharmaceutical Sciences. Mack Publishing Company, 1995
- **BARETTI M et al.** *Pharmacol Ther.*, 2018, vol. 189, 45-62
- **J. SAMBROOK et al.** Guide to Molecular Cloning Experiments. Science Press
- **ACIERNO et al.** Affinity maturation increases the stability and plasticity of the Fv domain of anti-protein antibodies. *J Mol Biol.*, 2007, vol. 374 (1), 130-46